# EUROPEAN PATENT APPLICATION

(11) **EP 4 800 694 A1**
(43) Date of publication of application: **02.09.2026**
(21) Application number: 25188670.1
(22) Date of filing: 10.07.2025
(51) Int. Cl.: G16H 10/20, A61B 5/16, A61B 5/00, G16H 20/70, G16H 50/30

(54) **PERSONALIZED DIGITAL THERAPEUTICS FOR SKILLS TRAINING USING ROLE-BASED INTERACTIONS**

(30) Priority: 27.02.2025 US 202519065633
(71) Applicant: Click Therapeutics, Inc., New York, NY 10013 (US)
(72) Inventor: Diaz Cortes, Margarita, New York, 10013 (US); Longenecker, Julia, New York, 10013 (US); Watson, Noreen, New York, 10013 (US)
(74) Representative: Marks & Clerk LLP

(57) **Abstract**

Various examples, systems, and methods are disclosed relating to a roleplay pipeline for treatment and skill development. One or more processors can receive, for a roleplay interaction having a defined role for a user and a defined role for a chatbot, content in the roleplay interaction. The one or more processors further can apply the content as input to at least one artificial intelligence (AI) model to cause the at least one AI model to generate an output for the chatbot. The one or more processors further can provide, via a chatbot interface, the output. The digital therapeutic application can improve treatment by providing structured roleplay interactions that support social engagement, strengthen emotion regulation, and improve skill development and can increase the efficacy of the medication that the user is taking to address social skills, emotion skills, behavior challenges, and/or cognitive processing deficits.

## Description

### CROSS-REFERENCE TO RELATED APPLICATION

The present application claims priority to and the benefit of U.S. Non-Provisional Application No. 19/065,633 titled "PERSONALIZED DIGITAL THERAPEUTICS FOR SKILLS TRAINING USING ROLE-BASED INTERACTIONS", and filed on February 27, 2025, which is incorporated herein by reference in entirety.

### BACKGROUND

Social skills, emotion skills, cognitive skills, and/or behavioral regulation deficits arise due to difficulties in assessing emotions, solving problems, verbal learning, and/or social perception. Common conditions associated with these deficits include autism spectrum disorder (ASD), social anxiety disorder, attention-deficit/hyperactivity disorder (ADHD), intellectual disabilities, developmental delays, schizophrenia, and/or among others. These deficits arise from a diverse array of factors, such as psychological conditions, behavioral challenges, genetic predispositions, and/or environmental influences.

Social skills and emotion skills deficits impact the ability to engage in meaningful social interactions, build and maintain relationships, maintain independence, achieve professional goals, and/or achieve overall well-being. Users with these deficits are at an increased susceptibility to cognitive difficulties, reduced self-confidence, feelings of inadequacy, mental health deterioration, and/or avoidance behaviors. Additionally, users with these deficits are at a greater risk of challenges in interpreting social cues, responding appropriately, resolving interpersonal conflicts, adapting to group dynamics, and/or maintaining conversations, which further reduces their ability to navigate real-world interactions. The overall quality of life for users with these deficits is often compromised and/or otherwise diminished.

At the cognitive level, social skills and emotion skills deficits lead to difficulties in areas such as processing social signals (e.g., facial expressions, tone of voice, body language, contextual cues) and performing tasks requiring verbal or social comprehension (e.g., asking for assistance, resolving conflicts, engaging in group discussions, managing workplace interactions). Specifically, individuals with social and emotional skill deficits often experience co-occurring executive functioning challenges, which can impact their ability to manage emotions, make decisions, organize thoughts, prioritize tasks, and/or engage in adaptive social behavior. These difficulties can interact and reinforce each other, further complicating social and emotional development. Furthermore, users with these deficits are at a heightened risk for developing secondary challenges (e.g., increased stress, isolation, difficulty maintaining employment, strained family relationships) and related impairments in daily functioning. For example, a failure to assess emotional cues in a conversation can lead to misunderstandings and reduced engagement in social or professional environments. In another example, an inability to handle constructive criticism can result in conflict avoidance. In yet another example, difficulty initiating conversations can prevent users from forming new connections.

Interventions for social skills and emotion skills deficits can be used to improve social functioning and reduce barriers to engagement. Improving these skills, however, is difficult due to ineffective personalization, lack of adaptability, insufficient feedback mechanisms, limited access to resources, and/or limited practice opportunities. In particular, a user's motivation to engage with interventions, as well as the inability of prior solutions to adapt to the user's specific needs, reduces the effectiveness of interventions. For example, the availability of group-based training can be restricted by financial and timing constraints; even within group-based training, the time allocated for personalized user goals within a single session is limited. Additionally, prior digital tools often fail to provide feedback that dynamically adjusts to user progress. Failing to incorporate personalized, adaptive mechanisms into these interventions can lead to inadequate skill development and reduced therapeutic outcomes.

### SUMMARY

Presented herein are systems and methods for providing personalized digital therapeutics to address social skills, emotion skills, cognitive processing deficits, and/or behavioral challenges. The digital therapeutic system described herein relates to using generative artificial intelligence (genAI) models in role-playing scenarios so users can practice their social skills and emotion skills in a safe virtual setting as part of a treatment plan, wherein the genAI model has the role of an individual that the user can interact with in a social setting (e.g., bus rider and bus driver, customer and coffeeshop barista, etc.). The genAI models (e.g., models for dynamic interaction adaptation, real-time role assignment, and/or compliance validation) can enhance user interactions during roleplay scenarios by generating realistic life-like outputs personalized to the specific user (e.g., User: "I'd like a cappuccino please." Chatbot: "Small, Medium, or Large? User: "Medium and no sugar." Chatbot: "Sure! Regular milk is fine?").

In some implementations, a chatbot can be powered by the AI models and can dynamically adjust the interactions based on a variety of factors, such as cognitive capacity, therapeutic focus areas, preferences (whether actively requested by the user or indirectly detected by the system based on analysis of user data over time), and/or availability of the user, ensuring that the sessions remain effective and tailored to user needs. By leveraging model(s), the system generates dynamic and personalized dialogue outputs that adjust in real-time to the performance of the user, helping users practice and refine these skills during sessions conducted through the digital therapeutic application. Additionally, traditional skills training programs are often generic and fail to meet the specific needs of users. Without such improved solutions described herein, users often face heightened stress, reduced confidence, lack of skills to successfully navigate real-world situations, and/or avoid certain daily life situations, compounding their disengagement from social contexts.

Specifically, this improved approach can use virtual interactions and roles with adaptive, real-time and/or near real-time feedback (e.g., direct feedback from the chatbot at the end of an interaction highlighting and/or identifying mood, tone, fluency, length, as examples, and/or indirect feedback by updating the responses and interactions by the chatbot during the course of the interaction with the user or subsequent interactions) to simulate real-world interactions, which functions to train multiple subdomains of social and cognitive skills (e.g., problem-solving, verbal communication, conflict resolution, self-regulation, emotion assessment, teamwork, and/or any interpersonal skill) simultaneously, improving progress through targeted practice in a controlled environment. The fear of making mistakes or experiencing negative consequences in real-life interactions presents a significant barrier to social engagement. The system described herein can mitigate this issue by providing a controlled environment where mistakes do not result in real-world repercussions. The personalized interaction model applies therapeutic goals to real-life-like situations relevant to the daily life of the user, thus helping to guide practical application of these skills in real-world contexts, leading to an enhanced clinical impact of these interventions and/or greater user engagement.

The benefit can be obtained by performing (e.g., continuously, periodically, and/or based on user progress) these interaction-driven and role-based activities together in cycles and/or user sessions, particularly in a structured, adaptive training program. Over time, for example, the fearbased responses of the user to social interactions can transition into confidence-driven behaviors focused on achieving desired personalized goals. Additionally, over time, in another example, users can develop the ability to generalize skills learned in one interaction to new, unpracticed interactions. Furthermore, over time, in yet another example, the system can identify emerging strengths and adjust training to refine higher-level skills. Thus, the interactions and roles build the skills of the user in assessing social cues and responding appropriately, while adaptive feedback mechanisms help refine these skills during interactions, personalize practice to user needs, and/or reinforce learned behaviors. For example, the system can guide a user through a simulated conversation to request assistance, resolve a conflict, build rapport, and/or set boundaries. The combination of structured interactions and adaptive feedback strengthens the ability of the user to navigate similar real-world situations.

The integration is an improvement over other prior intervention techniques that focus on generic conversational training or broad therapeutic exercises, without the integration of real-world applicability and/or adaptive personalization. If there is an imbalance between an underdeveloped social skills system and a need for emotion regulation, the roles and/or interaction-based activities disclosed herein can improve capacity and efficiency of social functioning through targeted, user-specific interventions. The combination of the roles and/or interaction-based training plans can target overlapping cognitive and social functions within the skill set of the user, involved in verbal learning, executive decision-making, and/or other examples, often improving the ability of the user to navigate daily interactions.

In addition, the digital therapeutic application described herein addresses the lack of personalized solutions that effectively target real-world social scenarios and adaptive progress tracking. The application uses structured, low-risk interactions with targeted feedback to translate improvements in skills such as problem-solving, active listening, and/or emotion assessment into practical outcomes. Based on the performance of the user during interactions, the system can dynamically adjust difficulty levels (e.g., language complexity, interaction length, interaction frequency, response variability) to further improve engagement and skill development. The digital therapeutic application can use user data (e.g., demographics, preferences, language fluency, personal goals, behavior indicators, session metrics, treatment history, cognitive capacity, conditions, and/or any specific therapeutic goals) to create personalized interactions (e.g., dialogues that can update and adapt to a given situation in the socio-environmental context of the user interactions) specifically for social skills, emotion skills, behavior challenges, and/or cognitive processing deficits of a user. Performance metrics generated from user performance (e.g., completion rates, progress indicators, success rates, response accuracy) can be presented to the user to encourage consistent engagement.

Accordingly, the digital therapeutic application addresses the prior lack of integrated interventions by providing digital interventions that dynamically adjust based on user responses to specific interactions and/or in specific roles. The application further provides a personalized approach to addressing social skills, emotion skills, behavioral challenges, and/or cognitive processing challenges that align with the specific needs and goals of each user. Through this integration of personalized therapeutic technical solutions, the efficacy of interventions targeting social skills, emotion skills, cognitive processing deficits, and/or behavioral challenges is improved, leading to better outcomes and overall quality of life for users using the system.

Additionally, the systems and methods described herein incorporate structured data sources and compliance validation models to generate outputs that align with ethical guidelines, contextual relevance, user preferences, linguistic appropriateness, cultural sensitivity, therapeutic alignment, and/or content safety (e.g., physical, emotional) standards, thereby addressing challenges associated with static, non-adaptive systems. By providing scalable and adaptable role-based interactions, the disclosed systems and methods improve computational efficiency, personalization capabilities, and dynamic feedback integration for applications such as skill-building platforms, interactive learning systems, and/or conversational interfaces.

Some implementations relate to a system including one or more processors coupled with memory. The one or more processors configured to receive, during a session for a roleplay interaction having a defined role for a user and a defined role for a chatbot, content from a user device in the roleplay interaction. The one or more processors configured to apply the content as input to at least one artificial intelligence (AI) model to cause the at least one AI model to generate an output for the chatbot based on the defined role for the user, the defined role for the chatbot, and the roleplay interaction. The one or more processors configured to provide, during the session via a chatbot interface, the output to the user device.

In some implementations, the one or more processors coupled with the memory is further configured to receive a request corresponding with initiating the session, identify the roleplay interaction from a plurality of roleplay interactions based at least on data of the user device, and initiate the session for the roleplay interaction. In some implementations, identifying the roleplay interaction from a plurality of roleplay interactions is based at least on a treatment journey and a condition of the user. In some implementations, a plurality of roleplay interactions are maintained in a data source and organized based on at least one interaction objective or at least one treatment outcome.

In some implementations, the defined role for the user is based on a condition of the user. In some implementations, the defined role for the chatbot is based on the roleplay interaction identified during an initiation of the session. In some implementations, the output corresponds to advancing a treatment journey corresponding with a condition of the user.

In some implementations, the output corresponds to providing a dialogue corresponding with completing an objective of the roleplay interaction or performing an action of the roleplay interaction. In some implementations, the one or more processors coupled with the memory is further configured to determine a metric of the session based at least on one or more (i) a plurality of user responses, (ii) data of the user device, (iii) content from the user device, (iv) interaction time, (v) completion of an objective of the roleplay interaction, or (vi) performance of an action of the roleplay interaction and update a profile of the user based on the metric. In some implementations, the one or more processors coupled with the memory is further configured to update, during the session, the defined role for the chatbot based on at least one of (i) a plurality of user responses, (ii) data of the user device, (iii) content from the user device, (iv) interaction time, (v) completion of an objective of the roleplay interaction or (vi) performance of an action of the roleplay interaction.

In some implementations, the roleplay interaction is personalized to the user based on at least one of data of the user device, content from the user device, completion of an objective of the roleplay interaction, performance of an action of the roleplay interaction, session data of the user, behavioral indicator of the user, demographic data of the user, values of the user, hobbies of the user, cognitive capacity of the user, preferences of the user, aesthetics of the user, reading level of the user, language level of the user, difficulty level of the user, complexity level of the user, or language of the user. In some implementations, the one or more processors coupled with the memory is further configured to apply the output as input to a compliance model to cause the compliance model to validate adherence of the output to at least one parameter of the chatbot.

In some implementations, the output includes a request for a response by the user device, and wherein the output is based on at least one of (i) performance during the session, (ii) selection of a new roleplay interaction, (iii) feedback generated during the session for the user, or (iv) feedback generated during the session from the user. In some implementations, the output includes feedback corresponding to at least one of (i) analysis of user performance or (ii) providing one or more recommendations for completing at least one objective of the roleplay interaction.

In some implementations, the at least one AI model is updated according to training data corresponding with a plurality of roleplay interactions. In some implementations, the one or more processors coupled with the memory is further configured to receive, during the session, a recording of the user and provide, during the session or after the session, at least a portion of the recording to the user.

In some implementations, the user is on a medication to address a social skill or emotion skills deficit at least in partial concurrence with the session for the roleplay interaction wherein the medication includes at least one of lithium, valproate, lamotrigine, carbamazepine, lamotrigine, chlorpromazine, fluphenazine, haloperidol, perphenazine, clozapine, olanzapine, ziprasidone, paliperidone, olanzapine, olanzapine, quetiapine, risperidone, aripiprazole, escitalopram, paroxetine, duloxetine, buspirone, fluoxetine, sertraline, venlafaxine, diazepine, lorazepam, alprazolam, clonazepam, nadolol, penbutolol, pindolol, propranolol, sotalol, timolol, acebutolol, esmolol, betaxolol, metoprolol, bisoprolol, labetalol, or carvedilol. The digital therapeutic application can improve treatment by providing structured roleplay interactions that support social engagement, strengthen emotion regulation, and/or improve skill development alongside pharmacological interventions and can increase the efficacy of the medication that the user is taking to address social skills of a user, emotion skills, behavior challenges, and/or cognitive processing deficits.

Some implementations relate to a method. The method including receiving, by one or more processors during a session for a roleplay interaction having a defined role for a user and a defined role for a chatbot, content from a user device in the roleplay interaction. The method including applying, by the one or more processors, the content as input to at least one artificial intelligence (AI) model to cause the at least one AI model to generate an output for the chatbot based on the defined role for the user, the defined role for the chatbot, and the roleplay interaction. The method including providing, by the one or more processors during the session via a chatbot interface, the output to the user device.

**In** some implementations, the method including receiving, by the one or more processors, a request corresponding with initiating the session, identifying, by the one or more processors, the roleplay interaction from a plurality of roleplay interactions based at least on data of the user device, and initiating, by the one or more processors, the session for the roleplay interaction. In some implementations, identifying the roleplay interaction from a plurality of roleplay interactions is based at least on a treatment journey and a condition of the user. In some implementations, a plurality of roleplay interactions are maintained in a data source and organized based on at least one interaction objective or at least one treatment outcome.

**In** some implementations, the defined role for the user is based on a condition of the user. In some implementations, the defined role for the chatbot is based on the roleplay interaction identified during an initiation of the session. In some implementations, the output corresponds to advancing a treatment journey corresponding with a condition of the user.

Some implementations relate to a method of treating or ameliorating a condition in a user in need of social skills or emotion skills training thereof. The method of treating or ameliorating the condition in the user in need of social skills or emotion skills training includes administering, to the user, a treatment including a digital therapeutic. The administering the digital therapeutic includes providing a session for a roleplay interaction, the roleplay interaction including a defined role for the user and a defined role for a chatbot. The administering the digital therapeutic includes applying content of the user as input to at least one artificial intelligence (AI) model to cause the at least one AI model to generate an output for the chatbot based on the defined role for the user, the defined role for the chatbot, and the roleplay interaction. The administering the digital therapeutic includes providing the output for the chatbot to a user device during the session via a chatbot interface.

In some implementations, administering the digital therapeutic to the user treats or ameliorates the condition in the user. In some implementations, administering the digital therapeutic further includes instructing the user to provide the content to the user device during the session.

In some implementations, administering the digital therapeutic further includes selecting the roleplay interaction for the session based on data of the user device. In some implementations, administering the digital therapeutic further includes updating, during the session, the defined role for the chatbot based on at least one of (i) a plurality of user responses, (ii) data of the user device, (iii) content from the user device, (iv) interaction time, (v) completion of an objective of the roleplay interaction or (vi) performance of an action of the roleplay interaction.

In some implementations, administering an effective amount of a medication to address a social skill or an emotional skill deficits, wherein the medication is selected from lithium, valproate, lamotrigine, carbamazepine, lamotrigine, chlorpromazine, fluphenazine, haloperidol, perphenazine, clozapine, olanzapine, ziprasidone, paliperidone, olanzapine, olanzapine, quetiapine, risperidone, aripiprazole, escitalopram, paroxetine, duloxetine, buspirone, fluoxetine, sertraline, venlafaxine, diazepine, lorazepam, alprazolam, clonazepam, nadolol, penbutolol, pindolol, propranolol, sotalol, timolol, acebutolol, esmolol, betaxolol, metoprolol, bisoprolol, labetalol, or carvedilol. The digital therapeutic application can improve treatment by providing structured roleplay interactions that support social engagement, strengthen emotion regulation, and/or improve skill development alongside pharmacological interventions and can increase the efficacy of the medication that the user is taking to address social skills of a user, emotion skills, behavior challenges, and/or cognitive processing deficits.

In some implementations, administering the digital therapeutic can further include determining a metric of the session based at least on one or more of (i) a plurality of user responses, (ii) data of the user device, (iii) content from the user device, (iv) interaction time, (v) completion of an objective of the roleplay interaction, or (vi) performance of an action of the roleplay interaction. In some implementations, administering the digital therapeutic can further include updating, during the session, the defined role for the chatbot based on at least one of (i) a plurality of user responses, (ii) data of the user device, (iii) content from the user device, (iv) interaction time, (v) completion of an objective of the roleplay interaction, or (vi) performance of an action of the roleplay interaction. In some implementations, administering the digital therapeutic can further include receiving, during the session, a recording of the user and providing, during or after the session, at least a portion of the recording to the user.

In some implementations, administering the digital therapeutic can further include adjusting the roleplay interaction during the session based on at least one of (i) therapeutic focus of the user, (ii) linguistic complexity of responses, (iii) behavioral indicators, or (iv) emotional engagement. In some implementations, administering the digital therapeutic can further include selecting the roleplay interaction from a plurality of roleplay interactions stored in a data source and organized based at least on one interaction objective or one treatment outcome. In some implementations, administering the digital therapeutic can further include providing, during the session, feedback to the user based on at least one of (i) performance during the roleplay interaction, (ii) completion of an objective, or (iii) engagement level of the user. In some implementations, administering the digital therapeutic can further include updating a user profile based on at least one of (i) content of the user, (ii) completion of an objective of the roleplay interaction, (iii) language level of the user, or (iv) session duration. In some implementations, administering the digital therapeutic can further include generating a progress report for the user based on at least one of (i) successful roleplay interactions, (ii) treatment milestones, or (iii) scenario completion rates. In some implementations, administering the digital therapeutic can further include applying the output of the chatbot as input to a compliance model to validate adherence of the output to at least one treatment guideline or therapeutic safety parameter. In some implementations, administering the digital therapeutic can further include modifying, during the session, the difficulty level of the roleplay interaction based on real-time analysis of user responses.

### BRIEF DESCRIPTION OF THE DRAWINGS

The present systems and methods for providing personalized digital therapeutics using role-based generative AI models are described in detail below with reference to the attached drawing figures, wherein:
FIG. 1 is a block diagram of an example of a system, in accordance with some implementations of the present disclosure;
FIG. 2 is a flow diagram of an example method for generating role-based interactions in a roleplay pipeline and treating and/or ameliorating a condition in a user, in accordance with some implementations of the present disclosure;
FIGS. 3A-3C are example interfaces for providing personalized digital therapeutics, in accordance with some implementations of the present disclosure;
FIG. 4 is a block diagram of an example server system and an example user device in accordance with an illustrative embodiment, in accordance with some implementations of the present disclosure.

### DETAILED DESCRIPTION

This disclosure relates to systems and methods for providing personalized digital therapeutics using role-based generative artificial intelligence (genAI) models to address social skills, emotion skills, behavior challenges, and cognitive processing deficits. For example, systems and methods in accordance with the present disclosure facilitate roleplay interactions with defined roles for a user and a chatbot during interactive sessions to improve cognitive and social skills. The systems can use content, such as free-text, audio, video, and/or other multimedia content from a user device to engage a chatbot in a roleplay interaction by applying as input to at least one artificial intelligence (AI) model configured to generate outputs for the chatbot based on predefined roles and objectives. The outputs of the AI model, provided via a chatbot interface, can guide users through personalized interactions designed to meet therapeutic goals, such as improving problem-solving, verbal communication, conflict resolution, emotion assessment, and/or social perception.

Some prior techniques for skills training rely on static scripts or preprogrammed responses, which lack personalization and adaptability to the unique needs of the user. These prior approaches fail to adjust to user progress or provide realistic and/or meaningful roleplay interactions. Additionally, such methods often lack structured interaction objectives and treatment outcomes, resulting in limited therapeutic efficacy. For example, prior systems cannot adapt to a demographic of the user, preferences of the user, and/or cognitive capacity of the user, and often do not address the technical importance for dynamic feedback during interactions. The systems and methods described herein overcome these limitations by generating and/or otherwise implementing roleplay interactions using genAI models, which provide personalized responses based on content, user data, user progress, demographics, interaction objectives, therapeutic goals, user preferences, behavior indicators, and/or session metrics.

Systems and methods in accordance with the present disclosure include receiving, during a session, content from a user device as input corresponding to a roleplay interaction involving a defined role for the user and a defined role for the chatbot. For example, the system can apply the content as input to at least one AI model to generate an output that corresponds to advancing and/or otherwise modifying the roleplay interaction. Outputs can be presented to the user device via a chatbot interface to maintain engagement, reinforce skill-building, and/or guide the user toward completing interaction objectives. Additionally, the system can dynamically adjust the defined role (e.g., customer service agent, friend, neighbor) of the chatbot during the session based on user responses, interaction time, interaction difficulty, interaction progress, and other examples described herein.

The system can also identify and initiate a session for a roleplay interaction from a plurality of interactions stored in a data source. For example, interactions can be selected based on demographics, preferences, language fluency, personal goals, behavioral indicators, session metrics, treatment history, cognitive capacity, conditions, emotional complexity, interaction length, role objectives, user engagement level, response adaptability, interaction progression structure, conversational tone, therapeutic goals, interaction objectives and/or treatment outcomes.

In some implementations, the system can update a user profile based on session metrics. Session metrics can include, for example, a plurality of user responses, interaction time, completion of interaction objectives, and/or performance during roleplay actions. The session metrics can be used to personalize future interactions and adjust difficulty levels to align with the progress of the user, build confidence, and/or therapeutic goals. For example, if a user demonstrates proficiency in one interaction, subsequent interactions can incorporate more complex and/or sophisticated interactions and/or higher language levels.

Additionally, the systems and methods include applying the chatbot output to a compliance model to validate adherence to predefined parameters, such as ethical guidelines, contextual relevance, user preferences, linguistic appropriateness, cultural sensitivity, therapeutic alignment, and/or content safety (e.g., physical, emotional) standards. For example, the compliance model can ensure that outputs do not include harmful content, misleading information, culturally insensitive remarks, and/or inappropriate content while maintaining the therapeutic objectives of the session. Outputs can also include feedback for the user, such as recommendations for completing interaction objectives or guidance on improving specific skills. Outputs can further include personalized reports summarizing user progress.

In some implementations, the system can receive a recording of the user during the session and provide at least a portion of the recording back to the user as feedback. For example, the user can review their recorded responses to identify areas for improvement, such as tone, clarity, and/or emotional expression. The system can also incorporate real-time and/or near real-time feedback into the session, guiding users toward more effective interactions. The roleplay interactions disclosed herein can be personalized based on a variety of user-specific factors, such as demographics, preferences, language fluency, personal goals, behavior indicators, session metrics, treatment history, cognitive capacity, conditions, and/or any specific therapeutic goals. For example, interactions can be personalized to align with the data of the user device, content from the user device, completion of an objective of the roleplay interaction, performance of an action of the roleplay interaction, session data of the user, behavioral indicator of the user, demographic data of the user, values of the user, hobbies of the user, cognitive capacity of the user, preferences of the user, aesthetics of the user, reading level of the user, language level of the user, difficulty level of the user, complexity level of the user, and/or language of the user. By integrating the personalization features, the system can ensure that at least one (e.g., each) session can be relevant and engaging for the user, increasing the likelihood of achieving therapeutic outcomes.

The systems and methods described herein improve upon traditional technical solutions by generating adaptive, role-based interactions that are personalized to the unique needs and goals of each user. By using genAI models and structured scenario frameworks (for example, provided by an administrator), the disclosed system provides an improved technical solution for improving social skills, emotion skills, behavioral challenges, and/or cognitive processing deficits. For example, the integration of real-time and/or near real-time feedback, compliance validation, and/or personalized interaction selection ensures that the therapeutic interventions remain aligned with user progress, resulting in improved outcomes and enhanced quality of life.

The systems and methods described herein can be used for a variety of purposes, including therapeutic training for users with social and emotion-related challenges, role-based learning for professional development, adaptive education programs, interactive coaching applications, and/or any adaptive skill-building platforms. Additionally, these methods can improve the accessibility and effectiveness of digital therapeutics, providing users with a controlled environment to practice and refine their skills.

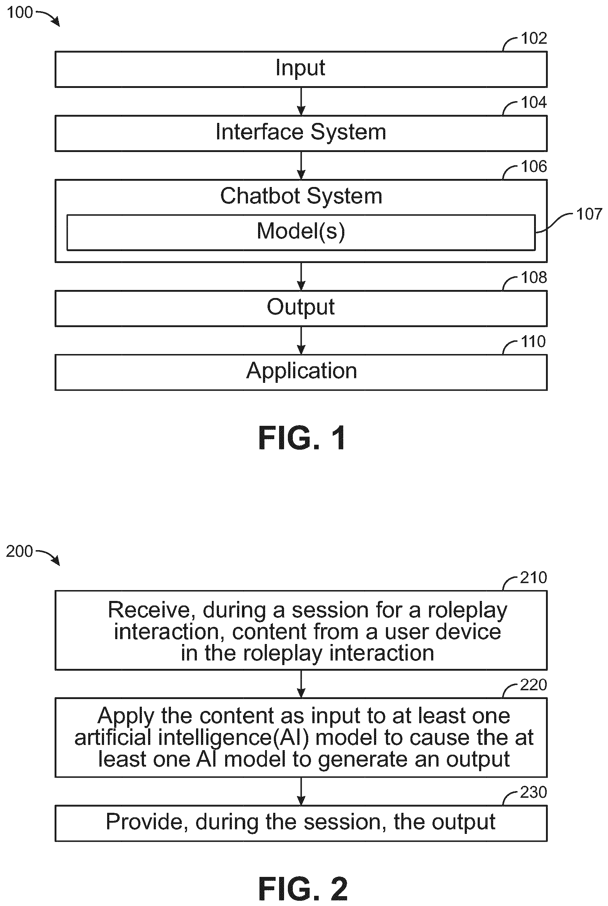

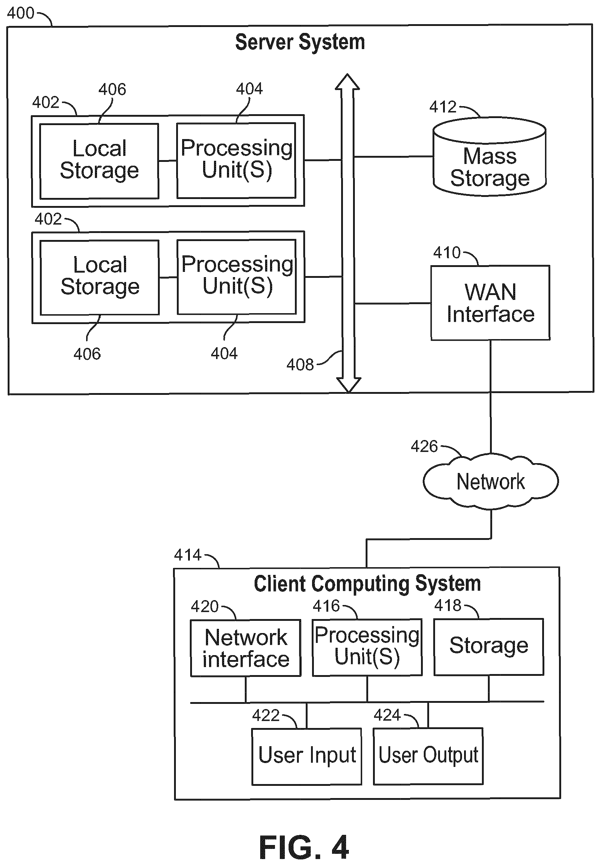

With reference to FIG. 1, FIG. 1 is a block diagram illustrating a system 100 in accordance with some implementations of the present disclosure. The system 100 includes components that can be implemented as discrete hardware, distributed components, and/or a combination of hardware, firmware, and/or software. The components of the system 100 can be configured to perform functions described herein, and the arrangement of these components can vary depending on the implementations. The arrangement shown in FIG. 1 is provided as an example and is not limiting. Other configurations and elements (e.g., machines, interfaces, functions, orders of operations, and/or groupings of functions) can be included in addition to and/or in place of those depicted. Some components can be omitted in particular implementations, depending on the operational requirements. Additionally, some functional entities in the system 100 can be grouped differently or implemented in alternative locations. The functions described in connection with the components of the system 100 can be performed by a processor and/or processors executing instructions stored in a memory. The instructions can direct the processor(s) to execute operations corresponding to one or more components of the system 100. The system 100 can also include interfaces or connections (e.g., wired and/or wireless communication technologies) that facilitate communication between components. The implementations can vary based on the requirements of the system and its operational environment. In some implementations, the systems, methods, and processes described herein can be executed using similar components, features, and/or functionality to those of example server system 400 of FIG. 4 and/or example user device 414 (depicted as "client computing system 414") of FIG. 4.

As used herein, "condition" refers to a symptom, disease, disorder, syndrome, impairment, deficit, dysfunction, abnormality, or any physiological, psychological, neurological, cognitive, behavioral, developmental, and/or emotional state that affects the health, functioning, and/or well-being of an individual. A condition can include temporary or chronic states, congenital or acquired impairments, progressive or episodic disorders, and/or any physiological or neurocognitive deviation from typical functioning that impacts an ability of an individual to engage in daily activities, social interactions, emotional regulation, cognitive processing, motor function, communication, and/or adaptive behavior. Example conditions that are treated in embodiments described herein include, but are not limited to, one or more of autism spectrum disorder (ASD), social anxiety disorder, attention-deficit/hyperactivity disorder (ADHD), intellectual disabilities, developmental delays, and/or schizophrenia.

As used herein, treatment, therapy, or therapeutic goals, outcomes, objectives, or challenges to overcome, are used interchangeably.

The system 100 can implement at least a portion of a roleplay pipeline, such as a therapeutic interaction pipeline, a behavioral training pipeline, or a skill development pipeline. The system 100 can be used to improve social interaction skills and/or enhance emotion assessment training by any of various systems described herein, including assertiveness training systems, emotion training systems, professional communication systems, adaptive language proficiency systems, social skills reinforcement systems, therapeutic intervention systems, role-based interaction platforms, and/or behavioral therapy support systems.

Generally, the roleplay pipeline can include operations performed by the system 100. For example, the roleplay pipeline can include any one or more of an interfacing stage, a modeling stage, and/or an outputting stage. Each stage of the roleplay pipeline includes one or more components of the system 100 that perform the functions described herein. In some implementations, one or more of the stages can be performed during the training of AI models. Additionally, one or more of the stages can be performed during the inference phase using the AI models (e.g., model(s) 107).

The system 100 (e.g., implementing the roleplay pipeline) can receive, during a session for a roleplay interaction having a defined role for a user and a defined role for a chatbot, content from a user device in the roleplay interaction. In some implementations, implementing the roleplay pipeline can include the system 100 applying the content, such as free-text, audio, video, and/or other multimedia content as input to at least one artificial intelligence (AI) model to cause the at least one AI model to generate an output for the chatbot based on the defined role for the user, the defined role for the chatbot, and the roleplay interaction. Additionally, implementing the roleplay pipeline can include the system 100 providing, during the session via a chatbot interface, the output to the user device. Thus, the roleplay pipeline can provide technical improvements over conventional therapeutic systems relying on static scripts, by dynamically adapting roleplay interactions based at least on content, user data, user progress, demographics, interaction objectives, therapeutic goals, user preferences, behavioral indicators, and/or session metrics.

In some implementations, the interfacing stage can be the stage in the roleplay pipeline in which the system 100 can receive user inputs and facilitate interaction with a chatbot. The system 100 can include at least one interface system 104. The interface system 104 can receive, during a session for a roleplay interaction having a defined role for a user and a defined role for a chatbot, content (e.g., the input 102) from a user device in the roleplay interaction. The interface system 104 can process and standardize user inputs for real-time and/or near real-time (e.g., within a time frame that facilitates immediate and/or nearly immediate system responses, such as milliseconds to a few seconds, accounting for data transmission, processing delays, user device constraints, network latency, and/or the computational complexity of AI models, where such responsiveness supports a seamless interaction experience) application in the session. For example, during the interfacing stage, the interface system 104 can capture and format the content to align with predefined session parameters. In some implementations, the interface system 104 can receive and/or otherwise interface with the user device by establishing a secure communication channel for data exchange. The content (e.g., input 102) can be queries, responses, comments, and/or contextual inputs provided by the user during the roleplay session; typed responses, speech-to-text conversions, session-specific phrases, and/or any other form of user interaction; or audio and/or video recordings, user annotations, prompts, sentiment data, timestamps, and/or any additional session-related data. For example, the interface system 104 can receive and/or otherwise obtain the input 102 from the user device by polling the device during the session or via a push notification system.

In some implementations, the interface system 104 can initiate a roleplay interaction between the system 100 and the user device responsive to receiving a request corresponding with initiating a session. The user device can include a digital therapeutic application (e.g., the application 110) that can communicate with the interface system 104 to initialize and/or configure roleplay sessions. For example, the request can be initiated by interacting with the digital therapeutic application. In this example, the interaction can be selecting an interaction from a predefined list, specifying a goal for the session, providing contextual information about the condition and/or treatment journey of the user, and/or any related configuration activity.

Additionally, the interface system 104 can identify the roleplay interaction from a plurality of roleplay interactions based at least on data associated with the user device (e.g., user inputs, system interactions, historical records, clinical data, real-time monitoring, external databases, professional assessments, third-party integrations, behavioral patterns, contextual factors, and/or any other relevant information). The interface system 104 can use data obtained from the user device (e.g., location data, time of day, user preferences, current device activity, and/or any ongoing session metrics), data analyzing such data (e.g., interaction trends, behavioral patterns), data related to a treatment journey (e.g., past sessions, current difficulties, focus areas such as practicing social interactions or improving emotional assessment, problem-solving skills, confidence-building exercises, and/or any additional therapeutic goals), a condition of the user, user demographics (e.g., age, language preferences, cultural background, profession, and/or educational level), and/or any additional profile attributes to select a roleplay interaction. For example, past sessions (e.g., of a treatment journey) can be used by the interface system 104 to select an interaction to build skills progressively (e.g., over a treatment journey). In another example, behavioral indicators and a condition of the user can be used by the interface system 104 to select an interaction that aligns with the therapeutic focus of the user.

Additionally, when multiple data points and/or elements can be used to select the roleplay interaction, the interface system 104 can prioritize based on predefined weights and/or hierarchical rules. The interface system 104 can resolve conflicting inputs or ambiguous data using a decision-making algorithm and/or function (e.g., machine learning models trained on data, predefined priority rules, user-specific preferences, heuristic-based ranking methods, and/or any optimization techniques). For example, the interface system 104 can use the most recent session data to prioritize relevant interactions for the immediate needs of the user. In another example, the interface system 104 can analyze trends across multiple sessions to suggest long-term goal-focused interactions.

In some implementations, the system 100 can store and/or otherwise maintain a list and/or data structure of roleplay interactions that can be personalized to different user demographics, preferences, language fluency, personal goals, behavior indicators, session metrics, treatment history, cognitive capacity, conditions, and/or therapeutic goals. For example, the list and/or data structure can include interactions categorized by demographics, preferences, language fluency, personal goals, behavioral indicators, session metrics, treatment history, cognitive capacity, conditions, emotional complexity, interaction length, role objectives, user engagement level, response adaptability, interaction progression structure, conversational tone, therapeutic goals, interaction objectives and/or treatment outcomes. In some implementations, the interface system 104 can generate roleplay interactions on demand and/or in real-time and/or near real-time by using generative AI models with predefined interaction templates (for example, based on social scenarios provided by an administrator) and/or user-specific inputs. For example, the interface system 104 can create variations of existing interactions to match user progress and/or therapeutic focus (e.g., of the treatment journey).

In some implementations, the plurality of roleplay interactions can be maintained in a data source (e.g., relational databases, NoSQL stores, cloud-based repositories, and/or any structured storage systems) and organized based on at least one interaction objective or at least one treatment outcome. The interaction objective can be, for example, successfully obtaining information from a bus driver about the quickest bus route to take. For example, a relational database can be organized based on interaction type and difficulty where at least one (e.g., each) can be tagged with skill categories and therapeutic outcomes. The treatment outcome can be improved confidence, improved communication, developed assertiveness, improved empathy, improved emotional assessment, reduced stress, improved confidence, improved skills to successfully navigate real-world situations, and/or any goal-based metric. For example, a NoSQL database can be organized based on treatment progress and user performance trends (e.g., towards achieving a treatment outcome) where interactions can be retrieved and ranked based on relevance to therapeutic milestones (e.g., of a treatment journey).

Generally, a roleplay interaction can be an interactive dialogue session designed to simulate real-world social and/or emotional interactions for therapeutic purposes. The roleplay interaction can be personalized and/or customized to mimic and/or imitate daily life situations, professional conversations, emotional interactions, family discussions, workplace exchanges, conflict resolution, and/or any other situation relevant to therapeutic objectives. The roleplay interaction can be a conversation.

In some implementations, prior to initiating the session for the roleplay interaction, the interface system 104 can determine a defined role for a user (e.g., patient, participant, trainee, customer, and/or any role aligned with the objective of the interaction) and determine a defined role for a chatbot (e.g., peer, authority figure, customer service representative, manager, friend, neighbor, and/or any role relevant to the condition or therapeutic goal). The defined role for the chatbot can be based on the roleplay interaction identified before, during, or after, an initiation of the session.

Both the defined roles and/or the roleplay interaction can be personalized to the user, and updated dynamically, based on a variety of factors, including a condition of the user (e.g., social stress, reduced confidence, lack of skills to successfully navigate real-world situations, communication deficits, emotional dysregulation), challenge of the user (e.g., difficulty initiating conversations, responding to social cues, and/or any cognitive or emotional barriers), at least one of data (e.g., session metrics, session history, therapeutic focus, interaction preferences) of the user device, content (e.g., user queries, contextual inputs, session-specific responses, audio, video, and/or other multimedia content from the user device), completion of an objective (e.g., successfully navigating a simulated interaction, achieving a therapeutic milestone in a treatment journey) of the roleplay interaction, performance of an action (e.g., responding empathetically, initiating a conversation, making a request) of the roleplay interaction, session data (e.g., timestamps, duration, interaction complexity) of the user, behavioral indicator (e.g., tone, sentiment, engagement level) of the user, demographic data (e.g., age, language preference, cultural background) of the user, values of the user, hobbies (e.g., outdoor activities, technology interests) of the user, cognitive capacity (e.g., verbal fluency, memory ability) of the user, preferences (e.g., short interactions, structured prompts) of the user, aesthetics (e.g., color scheme, accessibility options) of the user, reading level (e.g., grade-level appropriate language, simplified text) of the user, language level (e.g., beginner, advanced, multilingual support) of the user, difficulty level (e.g., simple, moderate, challenging interactions) of the user, complexity level (e.g., single-step, multi-step interactions) of the user, and/or language (e.g., English, Spanish, French) of the user. Thus, it should be understood that roleplay interactions can adapt dynamically to meet user-specific therapeutic needs. The interface system 104 can monitor (e.g., continuously, periodically) and update interactions based on real-time and/or near real-time user progress.

Additionally, determining the defined roles and/or roleplay interaction can occur by analyzing the user profile and interaction requirements in real-time and/or near real-time. For example, the interface system 104 can select roles and/or a roleplay interaction dynamically based on past performance metrics. In another example, the interface system 104 can assign roles and/or a roleplay interaction based on predefined templates personalized to specific therapeutic objectives. Additionally, defining the roles and/or a roleplay interaction can include customizing dialogue prompts, behavioral guidelines, and/or interaction objectives for both the user and chatbot roles. For example, the interface system 104 can generate role-specific instructions to ensure alignment with session goals.

Additionally, when multiple data points and/or elements (e.g., the various metrics and data used to personalize the roleplay interaction) can be used to select the roleplay interaction, the interface system 104 can prioritize inputs based on relevance and predefined weights. The interface system 104 can utilize a scoring system to rank interactions according to their alignment with the user's goals, preferences, or other factors. For example, the interface system 104 can prioritize interactions that address identified skill gaps or therapeutic objectives in a treatment journey. In another example, the interface system 104 can combine performance data with current user inputs to recommend interactions that balance challenge and engagement.

The user device providing the content can be operating and/or otherwise implementing a digital therapeutic application (e.g., mobile app, web-based platform, desktop software, and/or any compatible user interface). In some implementations, the user device can be a smartphone, tablet, laptop, desktop computer, wearable device, and/or any internet-configured device. The user device can serve as the medium for accessing and interacting with the roleplay interactions. For example, the user device can allow real-time and/or near real-time data exchange between the user and the interface system 104 during sessions.

Additionally, the digital therapeutic application (e.g., the application 110) can be configured to provide an intuitive and engaging user interface personalized to the user (e.g., therapeutic goals, treatment journey, user preferences, cognitive capacity, and/or any relevant demographic or behavioral data). In some implementations, the digital therapeutic application can provide and/or otherwise administer treatment by guiding users through roleplay sessions, offering real-time feedback, and tracking progress.

The interface system 104 can interface with the digital therapeutic application (e.g., the application 110) to obtain input 102 by establishing secure API connections and/or communication protocols. The interface system 104 can facilitate integration between the application and the roleplay pipeline. For example, the digital therapeutic application can interface with the interface system 104 by sending user inputs and receiving chatbot outputs responsive to session activities and/or preconfigured triggers. In another example, the digital therapeutic application can interface with the interface system 104 by transmitting data obtained during a session, such as session metrics or performance metrics, responsive to real-time and/or near real-time activity or updates. Additionally, the interface system 104 can access and/or otherwise monitor the digital therapeutic application by analyzing session progress, tracking user activity, and/or analyzing interaction patterns. The interface system 104 can ensure the application 110 adheres to therapeutic objectives and provides appropriate content. For example, the interface system 104 can review data to recommend next steps for user improvement. In another example, the interface system 104 can monitor user engagement to update difficulty level, update roleplay interactions, and/or update interaction frequency.

In some implementations, the modeling stage can be the stage in the roleplay pipeline in which the system 100 can apply user inputs and predefined parameters to dynamically generate personalized chatbot responses. The system 100 can include at least one chatbot system 106. The chatbot system 106 can apply the content as input to at least one artificial intelligence (AI) model (e.g., the model(s) 107) to cause the at least one AI model to generate an output 108 for the chatbot based on at least one of the defined role for the user, the defined role for the chatbot, and/or the roleplay interaction. The chatbot system 106 can process content and simulate conversations and other real-life interactions that align with the therapeutic goals and/or objectives of the session. The output 108 corresponds to advancing a treatment journey corresponding with a condition of the user. For example, advancing the treatment journey can include helping the user progress in their therapeutic and/or skills plan. For example, during the modeling stage, the chatbot system 106 can evaluate user inputs to determine appropriate dialogue, refine prompts, ensure session engagement, adjust response complexity, tailor interaction frequency, incorporate personalized feedback, and/or any adaptive interaction adjustments. In some implementations, the chatbot system 106 can apply and/or otherwise generate an output 108 by using generative AI models trained on a corpus of user data, and/or roleplay interactions and corresponding metadata.

The generating of the output 108 can include constructing responses that guide users toward completing defined objectives and/or goals of the session (e.g., during a treatment journey) while adhering to parameters (e.g., ethical guidelines, contextual relevance, user preferences, linguistic appropriateness, and/or content safety standards). The output 108 for the chatbot can include providing a dialogue corresponding with completing an objective (e.g., initiating a conversation, clarifying a misunderstanding, practicing empathetic responses, expressing emotions effectively, improving conversational flow, among others) of the roleplay interaction or performing an action (e.g., responding to a query, requesting assistance, making a suggestion, resolving a conflict, articulating a request, among others) of the roleplay interaction. For example, the chatbot system 106 can generate a step-by-step dialogue to help a user practice asking for help with a task, such as borrowing a ladder from a neighbor. In some implementations, the output can include a request for a response by the user device (e.g., the application 110). The request can be a prompt for additional information, clarification, and/or a follow-up action. For example, the request can be a question about the preferences of the user for continuing the interaction. In another example, the request can be a suggestion for refining a response to align with the interaction objective.

In addition to providing a dialogue corresponding with completing an objective of the roleplay interaction or performing an action of the roleplay interaction or requesting a response, the chatbot system 106 can output dialogue suggestions, roleplay prompts, progress metrics, and/or completion indicators (e.g., contextualized feedback, session summaries, interaction transitions, and/or any performance-based recommendations). In one example, the output 108 can be a summary of the performance of the user with recommendations for improvement (e.g., feedback).

Additionally, the output can be based on at least one of (i) performance during the session (e.g., how well the user engages or responds in the current session), (ii) selection of a new roleplay interaction (e.g., choosing another simulated activity based on user progress or session data), (iii) feedback generated during the session for the user, (iv) feedback generated during the session from the user, (v) real-time user engagement, and/or (vi) therapeutic alignment. For example, the model(s) 107 can generate the output for the chatbot based on performance during the session where the output can be a personalized prompt for deeper engagement or skill refinement. In another example, the model(s) 107 can generate the output for the chatbot based on selection of a new roleplay interaction where the output can be a transition to an interaction aligned with the therapeutic needs of the user.

In some implementations, the output can include feedback corresponding to at least one of (i) analysis of user performance or (ii) providing one or more recommendations for completing at least one objective of the roleplay interaction. For example, the feedback can include real-time and/or near real-time suggestions for improving responses or aligning them with interaction goals. For example, the chatbot can suggest rephrasing a response to enhance clarity or empathy. Additionally, feedback corresponding to providing one or more recommendations for completing at least one objective of the roleplay interaction can include step-by-step guidance for achieving an interaction milestone or overcoming specific challenges. For example, the chatbot can recommend focusing on specific conversational strategies to resolve a simulated conflict effectively.

In some implementations, the chatbot system 106 can be configured (e.g., trained, updated, fine-tuned, has transfer learning performed, etc.) based at least on the training data of the at least one training dataset (e.g., roleplay session data, linguistic patterns, user progress metrics, behavioral responses, user interaction data, annotated therapeutic goals, among others). For example, one or more example user inputs and/or interaction templates of the training data can be applied (e.g., by the system 100, or in a pre-training process performed by the system 100 or another system) as input to the chatbot system 106 to cause the model(s) 107 to generate an estimated output. The estimated output can be evaluated and/or compared with annotated target outputs and/or predefined therapeutic responses of the training data that correspond with the one or more example user responses and/or interaction outcomes, and the model(s) 107 of the chatbot system 106 can be updated based at least on the differences and/or success indicators. For example, based at least on an output of performance evaluation metrics, one or more parameters (e.g., weights and/or biases) of model(s) 107 of the chatbot system 106 can be updated.

**In** some implementations, the chatbot system 106 can determine a metric of the session. The metric of the session can measure if the session was successful by scoring the performance of the user. A session can begin when the user initiates a roleplay interaction (or is asked to initiate a roleplay interaction), selects an objective, and/or provides input to the chatbot, and a session can end when an objective is completed, the user exits the session, the chatbot provides an output, and/or a predefined time limit is reached. The metric can be determined based at least on one or more of: (i) a plurality of user responses, (ii) data of the user device, (iii) content from the user device, (iv) interaction time, (v) completion of an objective of the roleplay interaction, and/or (vi) performance of an action of the roleplay interaction. For example, the chatbot system 106 can determine an engagement score (e.g., metric) based on a combination of the plurality of user responses, interaction time, and completion of an objective of the roleplay interaction. In this example, the score can quantify the ability of the user to follow prompts, respond appropriately, and/or achieve interaction goals. Additionally, the chatbot system 106 can update a profile and/or account of the user based on the metric. The user can have an account and/or progress record that can store session performance, therapeutic milestones, and/or user preferences. The profile and/or account can be updated in real-time and/or near real-time and/or after session completion. For example, the profile and/or account can be created by analyzing the initial assessment of the user, a treatment journey, medical records, and/or onboarding data. In this example, the profile can be further updated by adding session-specific metrics and/or behavioral observations.

**In** some implementations, the chatbot system 106 can update, during the session, the defined role for the chatbot based on at least one or more of: (i) a plurality of user responses, (ii) data of the user device, (iii) content from the user device, (iv) interaction time (e.g., duration of user engagement with the chatbot), (v) completion of an objective of the roleplay interaction, and/or (vi) performance of an action of the roleplay interaction. For example, the chatbot system 106 can update the defined role of the chatbot dynamically based on user input or interaction progress. For example, the chatbot system 106 can update the defined role from customer service agent to technical specialist when the user escalates an issue during a simulated interaction. In another example, the chatbot system 106 can update the defined role from peer to mentor when the user completes an interaction requiring social or emotional guidance.

In some implementations, the chatbot system 106 can apply the output of a first AI model (e.g., the model(s) 107) as input to a compliance model (e.g., a second AI model of the model(s) 107 trained and/or implemented to validate ethical guidelines, contextual relevance, user preferences, linguistic appropriateness, cultural sensitivity, therapeutic alignment, and/or content (e.g., physical, emotional) safety standards) to cause the compliance model to validate adherence of the output to at least one parameter. In some implementations, the compliance model can monitor interactions for language indicative of self-harm or harm to others. If such language is detected, the system 100 can trigger a pop-up notification providing a suicide prevention hotline number and/or an option to dial 911 directly from the application, providing immediate access to crisis support resources. The compliance model can validate the chatbot output of the AI model is compliant with predefined rules for behavior and interactions. For example, the chatbot system 106 can implement the model(s) 107 to perform compliance on outputs of other model(s) 107 such that the dialogue generated aligns with session goals while mitigating and/or reducing risks of inappropriate or harmful content.

In some implementations, the outputting stage can be the stage in the roleplay pipeline in which the system 100 can deliver interaction-specific responses to the user based on inputs and defined roles. The system 100 can include at least one chatbot system 106. The chatbot system 106 can provide, during the session via a chatbot interface, the output 108 to the user device (e.g., operating an application 110). The chatbot system 106 can generate and send outputs to guide the user in completing objectives or advancing a roleplay interaction. For example, during the outputting stage, the chatbot system 106 can transmit dialogue and/or recommendations aligned with the roleplay interaction objectives and/or goals.

The application 110 can be installed and/or otherwise maintained by the user device (e.g., mobile phone, tablet, wearable device, desktop computer, smart speaker, and/or any compatible hardware). The application 110 can receive the output 108 for presentation, logging session data, triggering notifications, and/or any additional user interactions. For example, the output 108 can be presented on the application 110 of the user device to facilitate continuing interactions by the user during the session for a roleplay interaction. In this example, the application can display chatbot dialogue with options for user responses (e.g., free-text or other content, selection of actionable graphical elements, voice commands, touch-based responses, and/or any interaction mechanisms). In another example, the output 108 can be a summary of session progress with recommendations for future actions. In this example, the user can review the feedback and apply suggestions to improve skills in subsequent sessions. The digital therapeutic application 100 can improve treatment by providing structured roleplay interactions that support social engagement, strengthen emotion regulation, and/or improve skill development alongside pharmacological interventions and can increase the efficacy of the medication that the user is taking to address social skills, emotion skills, behavioral challenges, and/or cognitive processing deficits.

In some implementations, the chatbot system 106 can provide and/or otherwise transmit the output 108 by a secure communication protocol, such as HTTPS or WebSocket connections. The chatbot interface can be used to facilitate communications between the system 100 and the user device. The chatbot interface can manage session interactions, display outputs, and/or receive user inputs. For example, the chatbot interface can allow real-time and/or near real-time dialogue exchange through a conversational UI and other actions for providing a digital therapeutic.

In another example, the chatbot interface can integrate multimedia content, such as video and/or audio. In this example, the chatbot system 106 can provide, during the session or after the session, at least a portion of a recording (e.g., audio responses, video sessions, annotated transcripts, and/or any user interaction logs) of the user. Hearing or seeing their own voice can help users improve self-awareness, recognize patterns in their speech, and/or refine their communication skills. For example, users can identify areas for improvement in tone, pacing, clarity, emotional expression, word choice, conversational flow, engagement level, and/or any communication-related attributes. Additionally, reviewing their own recorded responses can reinforce learning by providing a means for self-assessment, allowing users to track their progress over time. In some implementations, the system 100 can include automated feedback mechanisms that analyze the recordings and provide structured recommendations to improve verbal and/or nonverbal communication strategies.

In some implementations, the chatbot system 106 can maintain, execute, train, update, and/or otherwise process, refine, or apply one or more artificial intelligence (AI) models during the modeling stage. In some implementations, the model(s) 107 can include any type of AI model capable of natural language processing, dialogue generation, and behavior modeling (e.g., transformer models, reinforcement learning models, and/or recurrent neural networks) to generate responses. For example, the model(s) 107 can be trained and/or updated to simulate role-specific interactions, improve accuracy of responses, and/or adapt to user progress, among other therapeutic improvements. The model(s) 107 can be or include a transformer-based model (e.g., a generative pre-trained transformer (GPT) model, a bidirectional encoder representation from transformers (BERT)). The machine-learning model(s) can be or include a sequence-to-sequence model, in some implementations. The chatbot system 106 can execute the model 107 to generate the output(s) 108. The chatbot system 106 can receive data (e.g., input 102 from interface system 104) to provide as input to the model(s) 107 from an administrator, which can include textual queries, contextual prompts, performance metrics, interaction parameters, and/or feedback data.

In some implementations, the chatbot system 106 can execute one or more AI models (e.g., the model(s) 107) by utilizing a training framework to improve the performance of the model 107 during the modeling stage. The framework can include implementing techniques such as gradient descent, backpropagation, and distributed training to roleplay large-scale datasets. The model(s) 107 can incorporate mechanisms such as regularization and weight pruning to maintain efficiency and prevent overfitting. For example, during execution, the chatbot system 106 can partition input data into mini-batches, apply loss functions, and update model parameters iteratively. The models 107 can support inference operations that include processing feature vectors, transforming raw input data (e.g., free-text, unstructured data, interaction-specific metadata, audio, video, other multimedia content, and/or any user data) and generating probabilistic predictions and/or metrics. The chatbot system 106 can integrate hardware accelerators such as GPUs or TPUs to manage high computational demands, for example when processing various roleplay interactions and/or performing real-time inference.

In some implementations, the chatbot system 106 can train and update AI models (e.g., the model(s) 107) through the roleplay pipeline that includes data preprocessing, feature engineering, and hyperparameter tuning. The preprocessing stage can include normalizing datasets, processing missing data, and/or augmenting inputs for training the model(s) 107. Feature engineering can include dimensionality reduction techniques, such as principal component analysis (PCA) and/or t-SNE. The model(s) 107 can incorporate attention mechanisms, activations, and/or layered architectures to facilitate learning. In some implementations, the chatbot system 106 can evaluate trained models using performance metrics (e.g., precision, recall, and/or F1 score) and/or any therapeutic success indicators, to determine readiness for deployment and/or inference operations.

In some implementations, the model(s) 107 can include an input layer, an output layer, and/or one or more intermediate layers, such as hidden layers, which can each have respective nodes. The model(s) 107 propagate input data through a multi-layer architecture to generate role-specific outputs. For example, the input layer processes user-provided content or contextual inputs. For example, the output layer generates interaction-specific dialogue or recommendations. For example, the intermediate layers perform computations such as embedding extraction, attention scoring, and/or context resolution.

In some implementations, the model(s) 107 can include a hierarchical architecture including an input processing layer, a feature transformation layer, and/or an output generation layer. At least one (e.g., each) layer can include a plurality of nodes or subcomponents configured to perform specific computations. The model(s) 107 can process input data by propagating it through the layers. For example, the input processing layer can prepare and standardize raw input data, extract initial features, and/or perform basic linguistic analysis. For example, the feature transformation layer can apply non-linear transformations or perform dimensionality reduction using operations such as matrix multiplication and activation functions. For example, the output generation layer can generate conversational responses, actionable suggestions, and/or performance feedback personalized to the roleplay interaction and/or the user.

In some implementations, the system 100 can configure (e.g., train, update, fine-tune, apply transfer learning to) the model(s) 107 by modifying or updating one or more parameters, such as weights and/or biases, of various nodes of the model(s) 107 responsive to evaluating estimated outputs of the model(s) 107 (e.g., generated in response to receiving training examples in a training dataset, such as a training dataset including roleplay session data, linguistic patterns, user progress metrics, behavior responses, user interaction data, annotated therapeutic goals, and/or any additional interaction-specific inputs). In some implementations, the model(s) 107 can be updated according to training data corresponding with a plurality of roleplay interactions (e.g., from performance of various interactions and/or exercises by the user or other similar users, such as users having the same condition). The model(s) 107 updates can reflect changing user needs and/or interaction complexities to improve dialogue accuracy and therapeutic relevance. The chatbot system 106 can be or include various neural network models, including models that can operate on or generate data including roleplay model(s), compliance model(s), personalization model(s), emotion detection model(s), dialogue adaptation model(s), and/or various combinations thereof.

The chatbot system 106 can include any one or more artificial intelligence models (e.g., machine learning models, supervised models, neural network models, deep neural network models), rules, heuristics, algorithms, functions, or various combinations thereof to perform operations including natural language understanding, dialogue generation, and dynamic role adaptation, such as interpreting user input, generating interaction-specific responses, and/or ensuring session objectives are met. The model(s) 107 can be a neural network and/or machine-learning (ML) model trained to simulate real-world interactions, generate adaptive dialogue, and/or align outputs with therapeutic objectives and/or goals.

Still referring to FIG. 1, system 100 provides an improved LLM-powered approach to personalized roleplay interactions. Generally, system 100 includes a generative AI supported chatbot that provides real-life-like skills training by using a set of pre-defined interactions as a starting point to further personalize before, during, and/or after user sessions. The role of the LLM in the chatbot can be to simulate a given character with defined characteristics and behavior (e.g., designed considering needs and challenges of people living with a condition in need of social skills, emotion skills, behavioral challenges, and/or cognitive processing deficits training) in order to accomplish a goal provided to or provided by the user. Additionally, the LLM can be used to accomplish this goal by using a chatbot to generate a dialogue while interacting with the content responses of the user following ethical guidelines, contextual relevance, user preferences, linguistic appropriateness, cultural sensitivity, therapeutic alignment, and/or content safety (e.g., physical, emotional) standards set in the underlying framework.

In some implementations, the genAI chatbot (e.g., implemented by chatbot system 106) can use a natural language processing (NLP) engine capable of understanding user queries expressed in various forms (e.g., free-text, audio, video, other multimedia forms). The model(s) 107 can perform tokenization to divide content into meaningful units, named entity recognition (NER) to identify specific entities like names or locations, and part-of-speech tagging to categorize words based on their syntactic roles. For example, the model(s) 107 can apply dependency parsing to analyze grammatical relationships between words in a sentence and sentiment analysis to determine the emotional tone of the input. In another example, the model(s) 107 can leverage semantic similarity analysis to match user queries with relevant interaction objectives or responses.

Additionally, the genAI chatbot can be capable of understanding user queries in multiple languages and can be capable of extracting relations, keywords, and/or patterns from the queries. In some implementations, the genAI chatbot can dynamically adapt to a language level according to the needs of the user, including cultural norms of that language, and can provide a stable interface (e.g., does not have software bugs, defects, errors) that provides reliable answers. Additionally, the outputs (e.g., output 108) can be generated by the model(s) 107 for a designated reading level (e.g., 6th grade reading level) or a particular dialect (e.g., when the output is audio). In some implementations, the roleplay interactions can be related to daily life situations but allows the user to practice or develop skills in a low-risk manner (e.g., the user is practicing their social skills in a mobile application with a chatbot as compared to a real-world scenario with other individuals who could react in an unpredictable manner).

In some implementations, the chatbot and/or roleplay interactions can be provided to attempt to improve independence, courage, dedication, cooperation, health, order, safety, self-care, connection, trust, loyalty, and/or kindness, among others. In some implementations, the model(s) 107 can provide outputs (e.g., audio and/or video) to attempt to improve emotion assessments (processing), problem solving, verbal learning memory, and/or social perception. In some implementations, the model(s) 107 can provide a maximum time of dialogue interaction under 5 minutes to maximize engagement and promote repetition. In some implementations, the chatbot can integrate with a personalization layer based on demographics (e.g., age, sex, profession, values, hobbies, among others), behavior indicators, session metrics, treatment history, cognitive capacity, any specific therapeutic goals, or any of the other factors already discussed related to personalization of the defined roles and/or roleplay interaction.

In some implementations, the application 110 can allow users to input queries in natural language. The application 110 can be user-friendly and can include additional features such as actionable items triggered by chat history. The application can display the answers to the user in a clear and understandable format. The digital therapeutic application 100 can improve treatment by providing structured roleplay interactions that support social engagement, strengthen emotion regulation, and/or improve skill development alongside pharmacological interventions and can increase the efficacy of the medication that the user is taking to address social skills, emotion skills, behavioral challenges, and/or cognitive processing deficits.

In some implementations, the system 100 can use genAI for therapeutic training, skills training, and/or in the context of social skills, emotion skills, behavioral challenges, and/or cognitive processing deficits training treatment. Unlike general-purpose LLMs, the system 100 provides simulated real-life situations for training people living with a need for social skills, emotion skills, behavioral challenges, and/or cognitive processing deficits training. This ensures higher accuracy and relevance within the scientific domain compared to models trained on broader generic internet text. For example, the model(s) 107 can provide training for people living with a need for social skills, emotion skills, behavioral challenges, and/or cognitive processing deficits training in the context of facing real-life situations in a safe and controlled digital environment, that lead to a therapeutic benefit over time.

Additionally, system 100 can serve as a bridge of abstract situations to face real-life-like experiences. In some implementations, the model(s) 107 can be utilized to address social skills, emotion skills, behavioral challenges, and/or cognitive processing deficits faced by users requiring social skills, emotion skills, behavioral challenges, and/or cognitive processing deficits training, particularly in navigating real-life interactions and situations, which can impact daily functioning and overall quality of life.

In some implementations, the technical problems that system 100 addresses include providing an effective and personalized solution for users requiring social skills, emotion skills, behavioral challenges, and/or cognitive processing deficits training. System 100 addresses the challenge of limited opportunities for users with a need for social skills, emotion skills, behavioral challenges, and/or cognitive processing deficits training to practice essential abilities, such as emotion assessments, problem-solving, verbal learning memory, and social perception. These skills are provided in a safe and controlled environment via system 100, which uses generative AI models (e.g., model(s) 107) and predefined interactions in its chatbot system 106 to simulate realistic social scenarios. Without such solutions, users often face heightened stress, have reduced confidence, lack skills to successfully navigate real-world situations, and/or avoid certain daily life situations, compounding their disengagement from social contexts.

The fear of making mistakes or experiencing negative consequences in real-life interactions presents a significant barrier to social engagement. System 100 mitigates this issue by providing a controlled environment where mistakes do not result in real-world repercussions. The chatbot system 106, operating via digital therapeutic application 304 (e.g., application 110), uses AI-generated outputs tailored to the defined role of the user, allowing users to build confidence through guided practice designed to mimic real-life situations.

System 100 also addresses the challenge of interpreting social cues, responding appropriately, and maintaining conversations for users with cognitive impairments or social difficulties. By leveraging model(s) 107, the system generates dynamic and personalized dialogue outputs that adjust in real-time to the performance of the user, helping users practice and refine these skills during sessions conducted through the application 110. Additionally, traditional skills training programs are often generic and fail to meet the specific needs of users. System 100 solves this limitation by providing personalized, generative AI-driven interactions stored in its database and executed dynamically based on user data, such as session history, preferences, and/or challenges. The integration of chatbot system 106, model(s) 107, and personalized interactions ensures that the training is adapted to the unique needs of the user, creating a safe, controlled digital environment for real-life skills practice, thereby effectively enhancing social interactions and emotional regulation.

System 100 addresses the challenge of creating a generative AI-based platform tailored for users with a need for social skills, emotion skills, behavioral challenges, and/or cognitive processing deficits training. Using model(s) 107, system 100 generates personalized roleplay interactions to address social skills, emotion skills, behavioral challenges, and/or cognitive processing deficits in a user, such as emotion assessments and problem-solving, while ensuring outputs are accurate and align with ethical guidelines, contextual relevance, user preferences, linguistic appropriateness, cultural sensitivity, therapeutic alignment, and/or content safety (e.g., physical, emotional) standards. The chatbot system 106 dynamically adapts to user inputs during sessions executed through application 304, ensuring that outputs do not provide harmful or misleading information. Developing this system can include addressing both the technical complexity of generative AI and for outputs to be personalized to the specific challenges of the user.

Generally, the system 100 can receive an input. A chatbot can be provided a system prompt that instructs it to adhere to the selected interaction and desired outcome of the interaction with the user. In another example, an administrator can provide a scenario as a prompt, and the system can generate a variety of potential different interactions to fit that scenario. The users can provide text dialogue, audio dialogue, video dialogue, and/or other multimedia content in order to respond to the chatbot and drive the interaction forward to achieve the desired outcome. In some implementations, user inputs are sterilized for PII before being sent to chatbot system 106. The model(s) 107 can output (e.g., output 108) responses to user inputs acting as a specific character (e.g., role) in the interaction; the output can be text dialogue, audio dialogue, video dialogue, or other multimedia content. Additionally, the outputs can be monitored with LLM built-in safety settings as well as additional content monitoring in an intermediate API. Thus, the system 100 provides users with a safe and controlled environment to practice their skills training to address the challenges described herein, such as improving the social functioning, independence, and/or overall quality of life for users with a need for social skills, emotion skills, behavioral challenges, and/or cognitive processing deficits training. The chatbot provides a safe and controlled environment for these users to practice a set of skills without fear of judgment or negative consequences.

Additionally, the system 100 can provide a personalized knowledge base (e.g., interactions). The chatbot can be tailored to the specific needs and/or goals of the user, providing a more personalized and effective learning experience. Additionally, the system 100 can facilitate repetition and/or practice skills focus. The chatbot allows for repeated practice of different daily life skills, which can help to build confidence and improve performance in real-life situations. By targeting specific skills like emotion assessment, problem-solving, verbal learning, and/or social perception, the system 100 can directly address areas of difficulty commonly experienced by users with a need for social skills, emotion skills, behavioral challenges, and/or cognitive processing deficits training. Additionally, the system 100 can provide real-time feedback. The chatbot adapts dynamically to the responses of the user, identifies areas for improvement, and adjusts the difficulty level, in response to the user's progress in prior sessions. Additionally, the system 100 can provide enhanced engagement. The interactive nature of the chatbot, coupled with its ability to adapt to the preferences and capacity of the user and provide real-time feedback, keeps users engaged and motivated throughout the training process.

Additionally, the system 100 can be accessible and flexible. The chatbot can be user-friendly, accessible and convenient to use whenever the user has time for it (on-demand use), allowing users to practice skills at their own pace and convenience. In some implementations, by designing the interactions to keep them short and with a reachable goal, it can also be nonoverwhelming and engaging. This design reduces barriers to conventional therapeutic methods and increases the likelihood of consistent engagement. Additionally, the system 100 can facilitate reduced stigma. By providing a private and anonymous platform for skills training, the chatbot helps reduce the stigma associated with seeking help for social challenges.

In some implementations, the system 100 can utilize NLP to understand user input in content form, allowing natural and dynamic conversations that mimic real-life interactions. This allows for more nuanced and realistic practice interactions. Additionally, the system 100 can use an AI model and/or underlying LLM to generate responses that align with ethical guidelines, contextual relevance, user preferences, linguistic appropriateness, and/or content safety standards, and/or that are coherent and/or appropriate to the interaction, providing users with realistic and meaningful training practice. Additionally, the system 100 can use a knowledge base (e.g., roleplay interaction database). The chatbot can access and/or otherwise draw from a curated database of interactions specifically designed to address social skills, emotion skills, behavioral challenges, and/or cognitive processing deficits by users with a need for such training. This ensures the training content is both relevant and therapeutically beneficial. In some implementations, the model(s) 107 can include a personalization layer where user demographics and preferences are integrated to further personalize the interactions and responses, creating a more tailored and effective learning experience.

In some implementations, the system 100 can be configured to handle regulatory considerations and other content monitoring. The model(s) can be updated based on various ethical guidelines, contextual relevance, user preferences, linguistic appropriateness, cultural sensitivity, therapeutic alignment, and/or content safety (e.g., physical, emotional) standards in the genAI and healthcare industries. In some implementations, the system 100 can provide a chatbot that adheres to data privacy and/or security protocols (e.g., HIPAA complaint). For example, the model(s) 107 can process user inputs locally on the user device to minimize data transmission, apply differential privacy techniques to anonymize data during training and inference, and/or validate outputs against compliance parameters to ensure sensitive information is not stored or disclosed improperly. Additionally, user data can be anonymized and encrypted. For example, the interface system 104 can apply encryption protocols to secure data transmission between the user device and system 100, anonymize user identifiers before storing data in a database, and use tokenization to replace sensitive information with unique placeholders during processing. In some implementations, the system 100 can be filtered in the frontend before presenting them to the user.

Additionally, system 100 incorporates safeguards to address ethical concerns associated with AI in healthcare. For example, compliance models within system 100 validate outputs generated by model(s) 107 to ensure alignment with predefined interaction rules. Privacy protections are implemented to secure user data, and/or bias mitigation techniques are applied to ensure equitable outputs. These measures allow system 100 to deliver a safe and ethical solution for training social skills, emotion skills, behavioral challenges, and/or cognitive processing deficits while addressing the sensitive requirements of the healthcare context.

In some implementations, chatbot system 106 can implement a plurality of operations. For example, a chatbot can be provided a system prompt that instructs it to adhere to the selected interaction and desired outcome of the interaction with the user. For example, users can provide free-text dialogue in order to respond to the chatbot and drive the interaction forward to the provided outcome. In this example, the user inputs can be sterilized for PII before being sent to LLM API (e.g., of model(s) 107). The chatbot system 106 can generate outputs (e.g., output 108). For example, the chatbot can be instructed and/or expected to respond to user inputs acting as a specific character in the interaction. For example, outputs can be monitored with LLM built-in safety settings as well as additional content monitoring in an intermediate API.

The model(s) 107 can be LLM-based and can be trained to generate conversations and dialogues, following instructions given in the prompt. In some implementations, the chatbot can be configured to understand and adapt the language level to the needs and/or preferences of the user to avoid understanding barriers. For example, the chatbot can be capable of interacting with users of different languages than English (e.g., Spanish, French, German, Italian, Portuguese, Chinese, Japanese) as well as a mixture of languages, including incorporation of cultural norms for users of that language. In some implementations, the system 100 can store a knowledge base. An interactions database can be personalized to people living with social skills, emotion skills, behavioral challenges, and/or cognitive processing deficits training needs, as discussed above in relation to factors that can be considered in personalizing the defined roles and roleplay interactions and selecting those roleplay interactions (matrix with relations and definitions). The structure of how each interaction relates to those factors can be used by the model(s) 107 to generate outputs.

In some implementations, the chatbot provided by chatbot system 106 can provide a friendly and/or accessible user-interface (e.g., considering high contrast, colorblind, low motion, and/or special needs). For example, the user can interact with the chatbot through adaptive text formatting, voice-based interaction, and/or gesture-based inputs while completing one interaction per day. In some implementations, the chatbot can stay in a role and/or character and can be implemented to keep the user in the right direction during the dialogue.

In some implementations, the chatbot system 106 can be configured to provide a speech-to-text feature that converts spoken language from the user into textual input for processing within the roleplay interaction. The speech-to-text feature can support multiple languages, dialect recognition, and adaptive accuracy based on user-specific speech patterns, pronunciation, pacing, respiration, phonation, articulation, resonance, prosody, pitch, jitter, shimmer, rhythm, pausing, mumbling, lisping, dysarthria, stuttering, understandable and/or clarity, background noise filtering, and/or contextual inference. Additionally, the feature can enhance accessibility for users with physical and/or cognitive impairments that make typing difficult, improve engagement by facilitating natural conversation flow, and/or provide real-time transcription for review and feedback.

In some implementations, the user can be provided real-time feedback about user performance (e.g., to solve the challenge and/or achieve the goal in the interaction). In some implementations, the chatbot system 106 can be configured to provide post-interaction tips on how the user can improve and/or provide assistance on questions about how to address a given interaction. In some implementations, the chatbot system 106 can be configured to provide Frequently Asked Questions (FAQ) integrated in the intelligence of the chatbot to answer questions of the user regarding the digital tool (e.g., data security in the chatbot, how the user's data is used, how does the chatbot work, among others).

In some implementations, the chatbot system 106 can be configured to allow users to record their answers as audio/video and receive feedback on the analysis of the recording and allow replaying of the recording to learn about their speech.

In some implementations, the chatbot system 106 can be configured to provide speech feedback for the user (e.g., about tone, pacing, among others). The chatbot system 106 can analyze speech patterns, pronunciation, pacing, respiration, phonation, articulation, resonance, prosody, pitch, jitter, shimmer, rhythm, pausing, mumbling, lisping, dysarthria, stuttering, understandable and/or clarity to generate feedback that enhances verbal communication skills and aligns with therapeutic objectives.

In some implementations, the chatbot system 106 can be configured to be trained on being ethical, for example, based on principles relevant in the healthcare domain. The chatbot system 106 can incorporate ethical AI principles, ensuring compliance with healthcare guidelines, minimizing harm, and/or preventing misinformation.

In some implementations, the chatbot system 106 can be configured to prioritize data privacy and/or security and/or implement bias detection and mitigation strategies in the development of the chatbot. The chatbot system 106 can employ encryption, anonymization, differential privacy techniques, and/or bias monitoring frameworks to safeguard user data.

In some implementations, the chatbot system 106 can be configured to ensure that the chatbot is used as a complementary tool to support and improve, not replace, human interaction. The chatbot system 106 can facilitate structured roleplay exercises that reinforce real-world social engagement while preserving the role of human practitioners, caregivers, and/or support networks in therapeutic interventions.

In some implementations, the chatbot system 106 can be configured to use LLMs and the knowledge base curated by scientific experts in the field of mental health, social skills, emotion skills, behavioral challenges, and/or cognitive processing deficits training, ensuring the accuracy, reliability, and ability of the chatbot to generate appropriate and helpful responses. The chatbot system 106 can integrate domain-specific knowledge, validated therapeutic techniques, and/or structured response models.

In some implementations, the model(s) 107 can include a human-in-the-loop to ensure quality and safety in different rounds of testing and validation.

In some implementations, various roleplay interactions can be generated, stored, and/or identified by the system 100. For example, the system 100, leveraging model(s) 107, can generate adaptive roleplay interactions with varying complexity, ranging from simple single-interaction tasks to multi-step processes, such as a job interviewing sequence involving multiple stages. The chatbot powered by model(s) 107 can dynamically adjust the length of interactions based on the cognitive capacity, therapeutic focus areas, availability of the user, and other factors as described herein, ensuring that the sessions remain effective and tailored to user needs. Additionally, the system 100 can incorporate therapeutic goals, such as emotional regulation, problem-solving, and/or verbal learning, into its knowledge base to enhance its applicability. The system 100 can integrate with electronic health records (EHRs) to further personalize interactions by aligning them with user data, thereby improving effectiveness. Moreover, the system 100 can extend its roleplay interactions to address other conditions, such as smoking cessation, migraines, multiple sclerosis (MS), atopic dermatitis, obesity, oncology, insomnia, acute coronary syndrome, by adapting its modules (e.g., roleplay interactions) to include content targeting these pathologies. Unlike general-purpose LLM-powered chatbots, which focus on open-ended interactions, the model(s) 107 in this approach are trained and/or implemented with a structured knowledge base of interactions, prompt guidelines, therapeutic goals, and/or other factors as described herein, to address unique social skills, emotion skills, behavioral challenges, and/or cognitive processing deficits faced by users.

Thus, the system 100 can be configured to address social skills, emotion skills, behavioral challenges, and/or cognitive processing deficits faced by users, with a focus on emotion assessment, problem-solving, verbal learning, and social perception. The system 100, utilizing model(s) 107, tailors roleplay interactions and responses to align with the unique needs and goals of users, ensuring targeted therapeutic outcomes. Through the integration of generative AI (genAI), the system 100 provides a personalized and adaptive experience, dynamically adjusting to user preferences, such as language needs, interaction complexity, and/or demographic differences, in real-time and/or near real-time. The accessibility of the chatbot, available via smartphones or computers with an internet connection, allows users to engage in training conveniently at their own pace and within their chosen environment. Additionally, the system 100 can create a safe and controlled space for practicing social interactions, mitigating fears of judgment or negative consequences, and/or empowering users to build confidence and refine their skills in a supportive setting. This ensures that the training process is not only effective but also user-centered and inclusive.

In some implementations, system 100 can implement and/or configure a self-reflection tool guided by model(s) 107 to analyze user interactions post-completion. The system 100 can process session data, including user responses, engagement metrics, and/or behavioral indicators, and other examples discussed herein, to generate insights regarding social and emotional skill development. For example, system 100 can evaluate conversation patterns, emotional tone, response timing, confidence levels, and other examples discussed herein, to provide structured feedback on user progress. In another example, system 100 can generate a comparison of past and present interactions, highlighting improvements in social perception, verbal memory, problem-solving, and/or emotion assessment. In yet another example, system 100 can output guided reflection prompts (e.g., "How did you feel about your response?" or "What would you do differently next time?") to encourage users to critically assess their interactions. In some implementations, system 100 can maintain a historical record of user progress, allowing for trend analysis and goal tracking over multiple sessions. Additionally, system 100 can adapt future roleplay based on self-reflection insights.

In some implementations, system 100 can support and/or otherwise implement multimodal interaction schemes for engaging with the generative AI model, allowing users to select different modes of interaction based on their needs, training focus, therapeutic preferences, and other factors discussed herein. The system 100 can facilitate text-based chatbot conversations, voice-based or video-based interactions, and/or avatar-driven simulations, where each mode can provide distinct engagement benefits. For example, system 100 can process content through chatbot system 106 for users preferring written exchanges. In another example, system 100 can utilize speech recognition and synthesis models to facilitate voice-based interactions for users seeking verbal communication practice. In yet another example, system 100 can generate avatar-driven roleplay simulations that include facial expressions, gestures, and dynamic responses to replicate face-to-face interactions. In some implementations, system 100 can allow users to transition between interaction modes dynamically, adjusting based on complexity, cognitive load, user comfort level, and other factors discussed herein. Additionally, system 100 can evaluate user performance across multiple interaction modes, refining feedback and/or adaptive recommendations accordingly.

In some implementations, system 100 can integrate genAI-generated environmental sounds into roleplay interactions to improve realism and immersion during interactions, for example by providing as output 108. That is, system 100 can synthesize ambient audio elements based on the selected scenario, dynamically adjusting soundscapes to reflect real-world conditions. For example, system 100 can generate the hum of a coffee machine and background chatter in a cafe scenario to simulate ordering from a barista. In another example, system 100 can create the sounds of ringing phones and muffled conversations for a workplace scenario involving customer service and/or professional discussions. In yet another example, system 100 can adjust sound intensity and layering based on user progress and interaction frequency and other factors discussed herein, ensuring an adaptive and non-distracting experience. In some implementations, system 100 can personalize soundscapes based on user sensitivity levels and other factors discussed herein, enabling volume control, audio filtering, and/or selective ambient noise reduction. Additionally, system 100 can incorporate auditory cues as contextual reinforcement, aligning background sounds with user responses to enhance situational awareness and engagement.

With reference to FIG. 2, an example flow diagram illustrating a method for generating role-based interactions in a roleplay pipeline and treating and/or ameliorating a condition in a user, in accordance with some implementations of the present disclosure. It should be understood that this and other implementations described herein are examples. Alternative configurations, elements (e.g., machines, interfaces, functions, orders, or groupings), and omissions are possible. Many elements are functional and can be implemented as discrete or distributed components, combined with others, and located in various configurations. Functions can be executed using hardware, firmware, and/or software, such as processors executing instructions stored in memory. For example, the systems, methods, and processes can use components and functionality similar to the server system 400 and client computing system 414 of FIG. 4.

In FIG. 2, each block of method 200 represents a computing process that can be performed using hardware, firmware, and/or software, such as processors executing memory-stored instructions. The method can also be implemented as computer-readable instructions on storage media, provided as a standalone application, a service, a microservice via an API, and/or a plug-in. Method 200 is described with reference to the system of FIG. 1 but can also be executed by any other system or combination of systems described herein.

### Systems and Methods for Generating Role-Based Interactions in a Roleplay Pipeline

FIG. 2 is a flow diagram showing a method 200 for receiving, applying, causing, generating, administering, and/or providing operations, in accordance with some implementations of the present disclosure. Various operations of method 200 can relate to improving the personalization and efficacy of digital therapeutic applications. Prior systems often rely on and/or use static scripts or generic algorithms, which can lead to limited adaptability and reduced therapeutic impact. The technological problems can arise when these prior systems fail to adjust dynamically to user-specific data, resulting in reduced engagement and ineffective outcomes. Method 200 of FIG. 2 can solve these technological problems by implementing AI-driven roleplay personalization, thereby improving user engagement and therapeutic efficacy.

The method 200, at block 210, includes receiving, during a session for a roleplay interaction having a defined role for a user and a defined role for a chatbot, free-text, audio, video, gesture inputs, biometric data, haptic feedback, physiological signals, sensor data, contextual metadata, and/or any user data from a user device in the roleplay interaction. For example, the processing circuits can receive multimedia data (e.g., video, audio, text, graphics, still images, and/or animation data) to be modeled. In some implementations, the processing circuits can receive a request corresponding with initiating the session. In some implementations, the processing circuits can identify the roleplay interaction from a plurality of roleplay interactions based at least on data (e.g., location data, time of day, device preferences, current device activity) to select a role-play interaction. In some implementations, the processing circuits can initiate the session for the roleplay interaction. In some implementations, identifying the roleplay interaction from a plurality of roleplay interactions is based at least on a treatment journey and a condition of the user. The processing circuits can select the roleplay interaction further based on past sessions of the user and the current challenges or focus areas of the user (e.g., practicing social interactions).

**In** some implementations, the plurality of roleplay interactions can be maintained in a data source and organized based on at least one interaction objective or at least one treatment outcome. Additionally, the defined role for the user can be based on a condition of the user. The processing circuits can personalize the role to the condition and/or specific challenges of the user. In some implementations, the defined role for the chatbot can be based on the roleplay interaction identified during an initiation of the session. In some implementations, the roleplay interaction is personalized to the user based on at least one of data of the user device, content from the user device, completion of an objective of the roleplay interaction, performance of an action of the roleplay interaction, session data of the user, behavioral indicator of the user, demographic data of the user, values of the user, hobbies of the user, cognitive capacity of the user, preferences of the user, aesthetics of the user, reading level of the user, language level of the user, difficulty level of the user, complexity level of the user, and/or language of the user.

The method 200, at block 220, includes applying the content as input to at least one artificial intelligence (AI) model to cause the at least one AI model to generate an output for the chatbot. The output can be based on the defined role for the user, the defined role for the chatbot, and the roleplay interaction. In some implementations, the output can correspond to advancing a treatment journey corresponding with a condition of the user. The output can attempt to help the user progress in their therapeutic and/or skills plan. In some implementations, the output can correspond to providing a dialogue corresponding with completing an objective of the roleplay interaction or performing an action of the roleplay interaction. Additionally, the processing circuits can update, during the session, the defined role for the chatbot based on at least one of (i) a plurality of user responses, (ii) data of the user device, (iii) content from the user device, (iv) interaction time, (v) completion of an objective of the roleplay interaction or (vi) performance of an action of the roleplay interaction. The processing circuits can update the role of the chatbot dynamically based on user input and/or interaction progress.

In some implementations, the processing circuits can apply the output as input to a compliance model to cause the compliance model to validate adherence of the output to at least one parameter of the chatbot. The processing circuits can validate the chatbot output of the AI model is compliant with predefined rules for behavior and interactions. In some implementations, the output can include a request for a response by the user device. For example, the output can be based on at least one of (i) performance during the session, (ii) selection of a new roleplay interaction, (iii) feedback generated during the session for the user, or (iv) feedback generated during the session from the user. In this example, the performance during the session can represent how well the user engages or responds in the current session. In this example, the selection of a new roleplay interaction can represent choosing another simulated activity based on user progress or session data. Additionally, the output can include feedback corresponding to at least one of (i) analysis of user performance or (ii) providing one or more recommendations for completing at least one objective of the roleplay interaction. In some implementations, the at least one AI model is updated according to training data corresponding with a plurality of roleplay interactions. The processing circuits can improve the AI model from performance of various interactions and/or exercises by the user.

The method 200, at block 230, includes providing, during the session via a chatbot interface, the output to the user device. For example, the chatbot interface can be a communication interface with a digital therapeutic application executed by the user device. In some implementations, the processing circuits can determine a metric of the session (e.g., measuring if the session was successful by scoring the performance of the user). For example, the metric can be based on at least one or more of (i) a plurality of user responses, (ii) data of the user device, (iii) content from the user device, (iv) interaction time, (v) completion of an objective of the roleplay interaction, or (vi) performance of an action of the roleplay interaction. Additionally, the processing circuits can update a profile of the user based on the metric. In some implementations, the processing circuits can receive, during the session, a recording of the user. Additionally, the processing circuits can provide, during the session or after the session, at least a portion of the recording to the user.

In some implementations, the user is on a medication to address social skills, emotion skills, behavioral challenges, and/or cognitive processing deficits at least in partial concurrence with the session for the roleplay interaction wherein the medication includes at least one of lithium, valproate, lamotrigine, carbamazepine, lamotrigine, chlorpromazine, fluphenazine, haloperidol, perphenazine, clozapine, olanzapine, ziprasidone, paliperidone, olanzapine, olanzapine, quetiapine, risperidone, aripiprazole, escitalopram, paroxetine, duloxetine, buspirone, fluoxetine, sertraline, venlafaxine, diazepine, lorazepam, alprazolam, or clonazepam. The digital therapeutic application can improve treatment by providing structured roleplay interactions that support social engagement, strengthen emotion regulation, and/or improve skill development alongside pharmacological interventions and can increase the efficacy of the medication that the user is taking to address social skills, emotion skills, behavioral challenges, and/or cognitive processing deficits.

### SYSTEMS AND METHODS FOR TREATING OR AMELIORATING A CONDITION IN USERS IN NEED OF SOCIAL SKILLS, EMOTION SKILLS, BEHAVIORAL CHALLENGES, AND/OR COGNITIVE PROCESSING DEFICITS TRAINING VIA ADMINISTRATION OF DIGITAL THERAPEUTICS

Method 200 and system 100 can be implemented and/or otherwise configured for providing therapeutic interventions, including delivering roleplay-based training interactions to address social skills, emotion skills, behavioral challenges, and/or cognitive processing deficits in a user, as part of a digital therapeutic for treating or ameliorating a condition in a user in need of social skills, emotion skills, behavioral challenges, and/or cognitive processing deficits training.

In further detail, the methods of treatment and/or amelioration of the conditions may include administering a digital therapeutic to the user. The condition may be a result of genetics, brain chemistry (e.g., changes in neurotransmitter levels like dopamine or serotonin), mental health disorders, or personality traits, among others. The user may be of any demographic or trait, such as by age (e.g., an adult (above age of 18) or late adolescent (between ages of 18-24)) or gender (e.g., male, female, or non-binary), among others.

Method 200 and system 100 can be implemented and/or otherwise configured for treating or ameliorating a condition in a user in need of social skills or emotion skills training. The method of treating or ameliorating a condition in the user can include administering, to the user, a treatment including a digital therapeutic. Administering the digital therapeutic can include providing a session for a roleplay interaction, the roleplay interaction including a defined role for the user and a defined role for a chatbot. Administering the digital therapeutic can include applying content of the user as input to at least one artificial intelligence (AI) model to cause the at least one AI model to generate an output for the chatbot based on the defined role for the user, the defined role for the chatbot, and the roleplay interaction. Administering the digital therapeutic can include providing the output for the chatbot to a user device during the session via a chatbot interface.

**In** some implementations, administering the digital therapeutic to the user treats or ameliorates the condition in the user. In some implementations, administering the digital therapeutic can further include instructing the user to provide the content to the user device during the session.

**In** some implementations, administering the digital therapeutic can further include selecting the roleplay interaction for the session based on data of the user device. In some implementations, administering the digital therapeutic can further include updating, during the session, the defined role for the chatbot based on at least one of (i) a plurality of user responses, (ii) data of the user device, (iii) content from the user device, (iv) interaction time, (v) completion of an objective of the roleplay interaction or (vi) performance of an action of the roleplay interaction.

**In** some implementations, administering the digital therapeutic can further include administering an effective amount of a medication to address a social skill or emotional skill deficit, wherein the medication is selected from lithium, valproate, lamotrigine, carbamazepine, lamotrigine, chlorpromazine, fluphenazine, haloperidol, perphenazine, clozapine, olanzapine, ziprasidone, paliperidone, olanzapine, olanzapine, quetiapine, risperidone, aripiprazole, escitalopram, paroxetine, duloxetine, buspirone, fluoxetine, sertraline, venlafaxine, diazepine, lorazepam, alprazolam, clonazepam, nadolol, penbutolol, pindolol, propranolol, sotalol, timolol, acebutolol, esmolol, betaxolol, metoprolol, bisoprolol, labetalol, or carvedilol.

In some implementations, administering the digital therapeutic can further include determining a metric of the session based at least on one or more of (i) a plurality of user responses, (ii) data of the user device, (iii) content from the user device, (iv) interaction time, (v) completion of an objective of the roleplay interaction, or (vi) performance of an action of the roleplay interaction. In some implementations, administering the digital therapeutic can further include updating, during the session, the defined role for the chatbot based on at least one of (i) a plurality of user responses, (ii) data of the user device, (iii) content from the user device, (iv) interaction time, (v) completion of an objective of the roleplay interaction, or (vi) performance of an action of the roleplay interaction. In some implementations, administering the digital therapeutic can further include receiving, during the session, a recording of the user and providing, during or after the session, at least a portion of the recording to the user.

In some implementations, administering the digital therapeutic can further include adjusting the roleplay interaction during the session based on at least one of (i) therapeutic focus of the user, (ii) linguistic complexity of responses, (iii) behavioral indicators, or (iv) emotional engagement. In some implementations, administering the digital therapeutic can further include selecting the roleplay interaction from a plurality of roleplay interactions stored in a data source and organized based at least on one interaction objective or one treatment outcome. In some implementations, administering the digital therapeutic can further include providing, during the session, feedback to the user based on at least one of (i) performance during the roleplay interaction, (ii) completion of an objective, or (iii) engagement level of the user. In some implementations, administering the digital therapeutic can further include updating a user profile based on at least one of (i) content of the user, (ii) completion of an objective of the roleplay interaction, (iii) language level of the user, or (iv) session duration. In some implementations, administering the digital therapeutic can further include generating a progress report for the user based on at least one of (i) successful roleplay interactions, (ii) treatment milestones, or (iii) scenario completion rates. In some implementations, administering the digital therapeutic can further include applying the output of the chatbot as input to a compliance model to validate adherence of the output to at least one treatment guideline or therapeutic safety parameter. In some implementations, administering the digital therapeutic can further include modifying, during the session, the difficulty level of the roleplay interaction based on real-time analysis of user responses.

In some implementations, administering the digital therapeutic can further include modifying, during the session, the difficulty level of the roleplay interaction based on real-time analysis of user responses. In some implementations, administering the digital therapeutic can further include determining a baseline measure of the user's condition before performing any sessions, wherein the baseline measure includes at least one of Structured Clinical Interviews for International Classification of Diseases (ICD-10 or ICD-11) diagnostic criteria, the Schedule for Affective Disorders (SADS), or any other diagnostic assessment. In some implementations, administering the digital therapeutic can further include assessing improvements over the baseline measure following administration of the digital therapeutic. In some implementations, administering the digital therapeutic can further include determining that amelioration is shown when there is an improvement in any evaluation criteria over the baseline measure.

**In** some implementations, administering the digital therapeutic can further include generating a progress report for the user based on at least one of successful roleplay interactions, treatment milestones, or scenario completion rates. In some implementations, administering the digital therapeutic can further include providing, during the session, feedback to the user based on at least one of performance during the roleplay interaction, completion of an objective, or engagement level of the user. In some implementations, administering the digital therapeutic can further include updating a user profile based on at least one of content of the user, completion of an objective of the roleplay interaction, or language level of the user. In some implementations, administering the digital therapeutic can further include applying the output of the chatbot as input to a compliance model to validate adherence of the output to at least one treatment guideline or therapeutic safety parameter.

In some implementations, administering the digital therapeutic can further include selecting the roleplay interaction from a plurality of roleplay interactions stored in a data source and organized based at least on one interaction objective or one treatment outcome. In some implementations, administering the digital therapeutic can further include instructing the user to provide content to the user device during the session. In some implementations, administering the digital therapeutic can further include updating, during the session, the defined role for the chatbot based on at least one of a plurality of user responses, data of the user device, content from the user device, interaction time, completion of an objective of the roleplay interaction, or performance of an action of the roleplay interaction.

In at least partial concurrence with the time instance or the sessions during which the user is administered the digital therapeutic, the user may be on a medication to address social skills, emotion skills, behavioral challenges, and/or cognitive processing deficits in a user. For example, the medication may include mood stabilizers, such as lithium, valproate, lamotrigine, carbamazepine, or lamotrigine, among others. In some aspects, the medication may include antipsychotics, such as chlorpromazine, fluphenazine, haloperidol, perphenazine, clozapine, olanzapine, ziprasidone, paliperidone, olanzapine, olanzapine, quetiapine, risperidone, or aripiprazole, among others. In some aspects, the medication may include antidepressants or antianxiety medications, such as escitalopram, paroxetine, duloxetine, buspirone, fluoxetine, sertraline, or venlafaxine, among others. In some aspects, the medication may include benzodiazepines, such as diazepine, lorazepam, alprazolam, or clonazepam. In some aspects, the medication may include beta-blockers, such as nadolol, penbutolol, pindolol, propranolol, sotalol, timolol, acebutolol, esmolol, betaxolol, metoprolol, bisoprolol, labetalol, or carvedilol, among others. The medication may be of an effective amount (e.g., dosage or frequency), which may be sufficient to effect positive outcomes of the condition (e.g., reduction in symptoms). The efficacy of the medication in terms of addressing the user's condition may be improved or enhanced from the concurrence with the intervention performed and monitored via the digital therapeutic application.

The methods of treating or ameliorating conditions in a user in need of social skills, emotion skills, behavioral challenges, and/or cognitive processing deficits training thereof may be performed by any components of the system 100, such as the chatbot system 106 or the interface system 104, among others. The method may include one or more processors digitally administering, to the user, a treatment including a digital therapeutic. The digital therapeutic may include providing a session for a roleplay interaction as described above with respect to system 100 and method 200. The roleplay interaction may include a defined role for the user and a defined role for a chatbot. Administering the digital therapeutic may also include applying content of the user as input to at least one artificial intelligence (AI) model to cause the at least one AI model to generate an output for the chatbot based on the defined role for the user, the defined role for the chatbot, and the roleplay interaction as described with respect to system 100 and method 200. The content (e.g., input 102) can be queries, responses, comments, and/or contextual inputs provided by the user during the roleplay session; typed responses, speech-to-text conversions, structured feedback, session-specific phrases, and/or any other form of user interaction; or audio and/or video recordings, user annotations, prompts, sentiment data, timestamps, and/or any additional session-related data.

Further, administering the digital therapeutic includes providing the output for the chatbot to a user device during the session via a chatbot interface. The output can be in a same or different format as the input (e.g., text, audio, video). The output can include feedback corresponding to at least one of (i) analysis of user performance or (ii) providing one or more recommendations for completing at least one objective of the roleplay interaction. For example, feedback can include real-time and/or near real-time suggestions for improving responses or aligning them with interaction goals.

In some aspects, administering the digital therapeutic includes instructing the user to provide the content to the user device during the session. In yet another aspect, administering the digital therapeutic further includes selecting the roleplay interaction for the session based on data of the user device. In yet another aspect, administering the digital therapeutic includes updating, during the session, the defined role for the chatbot based on at least one of (i) a plurality of user responses, (ii) data of the user device, (iii) content from the user device, (iv) interaction time, (v) completion of an objective of the roleplay interaction or (vi) performance of an action of the roleplay interaction.

A baseline measure may be (e.g., by a user device) obtained prior to performing any sessions via a digital therapeutic application (e.g., the application). The baseline measure may indicate the condition of the user with respect to the severity of the condition. The baseline metric may include, for example, Structured Clinical Interviews for International Classification of Diseases (ICD-10 or ICD-11) diagnostic criteria and the Schedule for Affective Disorders (SADS), Composite Interview Diagnostic Interview (CIDI), General Behavior Inventory (GBI), Hypomanic Personality Score (HPS), Bipolar Spectrum Diagnostic Scale, Young Mania Rating Scale, Bech-Rafaelsen Mania Rating Scale, Altman Self-Rating Mania Scale, Self-Report Manic Inventory, Mood Disorder Questionnaire (MDQ), Patient Health Questionnaire (PHQ-9), Clinical Assessment Interview for Negative Symptoms (CAINS), Brief negative Symptom Scale (BNSS), Positive and Negative Symptom Scale (PANSS), Scale for the Assessment of Positive Symptoms (SAPS), Scale for the Assessment of Negative Symptoms (SANS), Negative Symptom Assessment - 16 (NSA-16), and Clinical Global Impression Schizophrenia (CGI-SCH), autism diagnostic observation schedule (ADOS), Autism Diagnostic Interview-Revised (ADI-R), Childhood Autism Rating Scale (CARS), Gilliam Autism Rating Scale (GARS) blood tests, genetic tests, or psychological screenings among others. Before performance of the intervention, the user may have an initial baseline metric satisfying a baseline threshold. The threshold may delineate, define, or identify a value for the initial metric at which the user is identified to be suitable or eligible for performance of the activities via the digital therapeutic application.

Administering the digital therapeutic to the user treats or ameliorates the condition in the user. In some aspects, treatment or amelioration of the condition may include an improvement over the baseline measure of any one or more of the baseline metrics following administration of the digital therapeutic. The method may include determining that amelioration is shown when there is an improvement in any evaluation criteria over the baseline metric following administration of the digital therapeutic. In some aspects, the amelioration may be determined (e.g., by the computing system or a clinician examining the user) to occur when the metric following administration is decreased from the baseline metric by a statistically significant margin. In some aspects, treatment or amelioration of the metric may include a decrease in symptoms following administration of the digital therapeutic. The decrease in symptoms may be established by the user's self-reported assessment of social skills, emotion skills, behavioral challenges, and/or cognitive processing deficits or an improvement in any one of the baseline metrics provided above.

### Example 1: Efficacy of Personalized Roleplay Interactions in a Digital Therapeutics to Target Conditions

With reference to efficacy of personalized roleplay interactions in digital therapeutics for social skills, emotion skills, behavioral challenges, and/or cognitive processing deficits training, a digital therapeutic application (e.g., application 110) incorporating AI model personalized roleplay interactions is provided to users with self-reported or HCP-diagnosed difficulties in social skills, emotion skills, behavioral challenges, and/or cognitive processing deficits. Participants may include users with autism spectrum disorder presenting communication challenges, users with generalized anxiety disorder impacting social interactions, users diagnosed with schizophrenia, and/or any other social and/or emotion interaction impairments, according to relevant diagnostic criteria.

In one example, the mobile app is provided to users in need of social skills, emotion skills, behavioral challenges, and/or cognitive processing deficits training. The study evaluates the efficacy, feasibility, safety, and/or acceptability of this application 110 in engaging users with roleplay-based interactions designed to simulate real-life interactions. This study spans approximately 2-52 weeks, with participation open to users aged 18 years and older. At least 20 participants are included, with the study structured across multiple testing rounds (e.g., rounds 1-52).

The study enrolls a minimum of 20 participants to ensure at least 10 completers. A subset of approximately 10 participants consent to participate in User Experience Research (UXR) interviews and surveys.

In at least partial concurrence with the time instance or the sessions during which the study participant is administered the digital therapeutic, the study participant may be administered a medication to address social skills, emotion skills, behavioral challenges, and/or cognitive processing deficits in a user. For example, the medication may include mood stabilizers, such as lithium, valproate, lamotrigine, carbamazepine, or lamotrigine, among others. In some aspects, the medication may include antipsychotics, such as chlorpromazine, fluphenazine, haloperidol, perphenazine, clozapine, olanzapine, ziprasidone, paliperidone, olanzapine, olanzapine, quetiapine, risperidone, or aripiprazole, among others. In some aspects, the medication may include antidepressants or antianxiety medications, such as escitalopram, paroxetine, duloxetine, buspirone, fluoxetine, sertraline, or venlafaxine, among others. In some aspects, the medication may include benzodiazepines, such as diazepine, lorazepam, alprazolam, or clonazepam. In some aspects, the medication may include beta-blockers. The digital therapeutic application can improve treatment by providing structured roleplay interactions that support social engagement, strengthen emotion regulation, and/or improve skill development alongside pharmacological interventions and can increase the efficacy of the medication that the user is taking to address social skills, emotion skills, behavioral challenges, and/or cognitive processing deficits.

Participants engage in a range of roleplay activities facilitated by the digital therapeutic application. The performance of participants and interaction data is analyzed according to clinical endpoints specified below.

Screening Period: All participants are screened for up to 7 days. Eligibility is determined based on inclusion and exclusion criteria, as described in the protocol. Assessments are conducted according to a Schedule of Activities and Assessments (SoA). Screening and Baseline Visits can occur on the same day. An interview design can be different for each round of interaction testing. In Round 0, the interview design can be virtual, given for a maximum duration of 1 hour and the user is provided a list of tests for specific prompts (e.g., on a form) to provide to the chatbot in application 110. In Round 1 and subsequent rounds, the interview design can be virtual for a maximum duration of 2 hours and the user can be provided a proof-of-concept tool with quantitative feedback.

Baseline Visit: At the conclusion of the Screening Period, eligible participants are enrolled and provided with the digital therapeutic application (e.g., application 110). Participants download and activate the application on their primary device, such as a smartphone, desktop computer, tablet, mobile device, and/or any computing device or system. They are instructed to engage with the application daily during the study. A baseline metric of the user's condition is obtained through screening of any one of the following assessments: Structured Clinical Interviews for International Classification of Diseases (ICD-10 or ICD-11) diagnostic criteria and the Schedule for Affective Disorders (SADS), Composite Interview Diagnostic Interview (CIDI), General Behavior Inventory (GBI), Hypomanic Personality Score (HPS), Bipolar Spectrum Diagnostic Scale, Young Mania Rating Scale, Bech-Rafaelsen Mania Rating Scale, Altman Self-Rating Mania Scale, Self-Report Manic Inventory, Mood Disorder Questionnaire (MDQ), Patient Health Questionnaire (PHQ-9), Clinical Assessment Interview for Negative Symptoms (CAINS), Brief negative Symptom Scale (BNSS), Positive and Negative Symptom Scale (PANSS), Scale for the Assessment of Positive Symptoms (SAPS), Scale for the Assessment of Negative Symptoms (SANS), Negative Symptom Assessment - 16 (NSA-16), and Clinical Global Impression Schizophrenia (CGI-SCH), autism diagnostic observation schedule (ADOS), Autism Diagnostic Interview-Revised (ADI-R), Childhood Autism Rating Scale (CARS), Gilliam Autism Rating Scale (GARS) blood tests, genetic tests, or psychological screenings among others

Engagement Period: Participants interact with the application daily, completing at least two roleplay sessions each week. The roleplay interactions are dynamically personalized based on data of the user device, content from the user device, completion of an objective of the roleplay interaction, performance of an action of the roleplay interaction, session data of the user, behavioral indicator of the user, demographic data of the user, values of the user, hobbies of the user, cognitive capacity of the user, preferences of the user, aesthetics of the user, reading level of the user, language level of the user, difficulty level of the user, complexity level of the user, and/or language of the user. Evaluations occur throughout the engagement period in accordance with a schedule of activities (SoA). Virtual interviews are conducted with approximately 10 participants at the end of various weeks (i.e.: Week 2, Week 4, and/or Week 6) to gather additional qualitative insights. Weekly surveys assess engagement and usability.

A structured study schedule, consistent with guidelines for evaluating digital therapeutics, organizes activities into multiple rounds. Each round involves virtual and/or in-person participation, with users engaging in activities such as completing roleplay interactions, providing free-text feedback, and responding to structured surveys. Study data is analyzed to refine the application, validate safety, and establish the efficacy of LLM-powered personalized roleplay interactions in addressing social skills, emotion skills, behavioral challenges, and/or cognitive processing deficits.

Follow-up Period: Following the engagement period, participants undergo post-treatment follow-up lasting 1-6 weeks. Evaluations focus on changes in social skills, emotion skills, behavioral challenges, and/or cognitive processing deficits, user satisfaction, and the application's perceived efficacy. Participants who participated in UXR interviews provide additional feedback during this phase. Evaluation metrics are obtained and compared with the baseline metrics associated with any one or more of the assessments provided above.

Primary Endpoints:
Engagement Metrics: Number of application usage days during the engagement period; Number of roleplay scenarios completed; Participant adherence to the protocol; Number of user responses per session; Average duration of engagement per session; Frequency of interaction with application prompts.
Efficacy Metrics: Changes in self-reported social confidence via a standardized scale; Changes in emotional regulation skills measured through in-app tasks; Changes in social engagement metrics (e.g., conversation initiation frequency); Improvements in perceived ability to handle real-life interactions; Reduction in anxiety scores during social interactions; Enhanced ability to follow conversational cues during roleplay. Changes in any one of the following assessments: Structured Clinical Interviews for International Classification of Diseases (ICD-10 or ICD-11) diagnostic criteria and the Schedule for Affective Disorders (SADS), Composite Interview Diagnostic Interview (CIDI), General Behavior Inventory (GBI), Hypomanic Personality Score (HPS), Bipolar Spectrum Diagnostic Scale, Young Mania Rating Scale, Bech-Rafaelsen Mania Rating Scale, Altman Self-Rating Mania Scale, Self-Report Manic Inventory, Mood Disorder Questionnaire (MDQ), Patient Health Questionnaire (PHQ-9), Clinical Assessment Interview for Negative Symptoms (CAINS), Brief negative Symptom Scale (BNSS), Positive and Negative Symptom Scale (PANSS), Scale for the Assessment of Positive Symptoms (SAPS), Scale for the Assessment of Negative Symptoms (SANS), Negative Symptom Assessment - 16 (NSA-16), and Clinical Global Impression Schizophrenia (CGI-SCH), autism diagnostic observation schedule (ADOS), Autism Diagnostic Interview-Revised (ADI-R), Childhood Autism Rating Scale (CARS), Gilliam Autism Rating Scale (GARS) blood tests, genetic tests, or psychological screenings among others
Usability Metrics: User satisfaction ratings (e.g., Likert-scale feedback on app usability); Qualitative feedback on the relevance and relatability of roleplay interactions; Feedback on the clarity of instructions provided in the app; Ratings on the ease of interaction with the chatbot interface; Feedback on the responsiveness of the application 110 to user input.
Safety Metrics: Absence of AI model hallucinations or unsafe responses during interactions; Participant-reported adverse experiences related to app usage; Analysis of content appropriateness for the target population; Evaluation of emotional triggers in roleplay interactions; Metrics of participant-reported discomfort during usage.

Exploratory Endpoints: Correlations between user engagement levels and perceived therapeutic outcomes; Analysis of roleplay interaction complexity versus user performance; Assessment of interaction relatability and user interest through qualitative feedback; Variations in performance metrics across demographic groups; Feedback on preferred roleplay scenario lengths; Correlations between user-reported satisfaction and measurable improvements in social confidence.

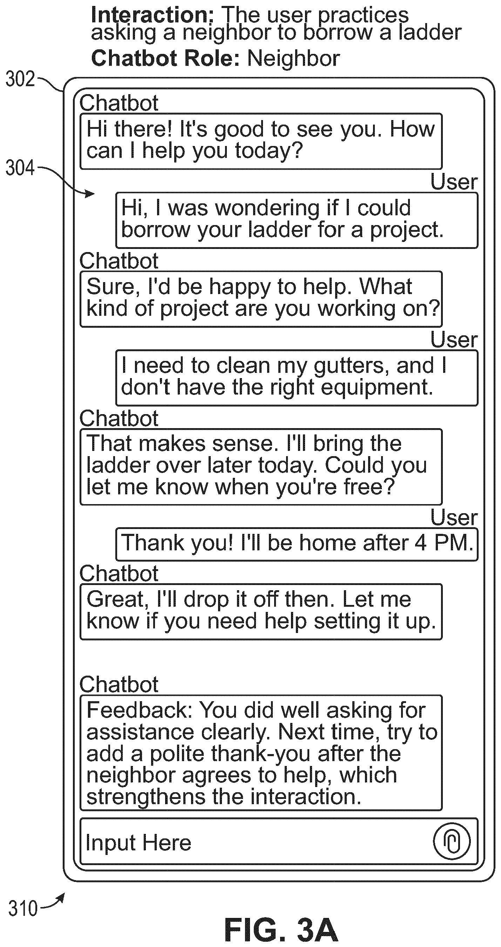

Referring now to FIG. 3A is an example interface for providing personalized digital therapeutics, in accordance with some implementations of the present disclosure. The system 100 interfaces with the digital therapeutic application 304 on the user device 302 to simulate the roleplay interaction 310. In this session, model(s) 107 are configured to process user input and generate chatbot outputs dynamically. The scenario involves the user practicing how to borrow a ladder from a neighbor. The chatbot begins, "Hi there! It's good to see you. How can I help you today?" The user inputs, "Hi, I was wondering if I could borrow your ladder for a project." The system 100 processes this input via model(s) 107, which generates a response: "Sure, I'd be happy to help. What kind of project are you working on?" The user continues, "I need to clean my gutters, and I don't have the right equipment." Model(s) 107 generate the reply of the chatbot: "That makes sense. I'll bring the ladder over later today. Could you let me know when you're free?" The user responds, "Thank you! I'll be home after 4 PM." The chatbot responds, "Great, I'll drop it off then. Let me know if you need help setting it up."

The system 100 evaluates user interaction data and provides feedback through the chatbot: "You did well asking for assistance clearly. Next time, try to add a polite thank-you after the neighbor agrees to help, which strengthens the interaction." The processing circuitry of system 100 uses model(s) 107 to analyze user input, refine the dialogue context, and/or assess task completion. Metrics such as input clarity and objectives can be tracked to personalize interactions and facilitate dynamic interaction generation and adaptive responses.

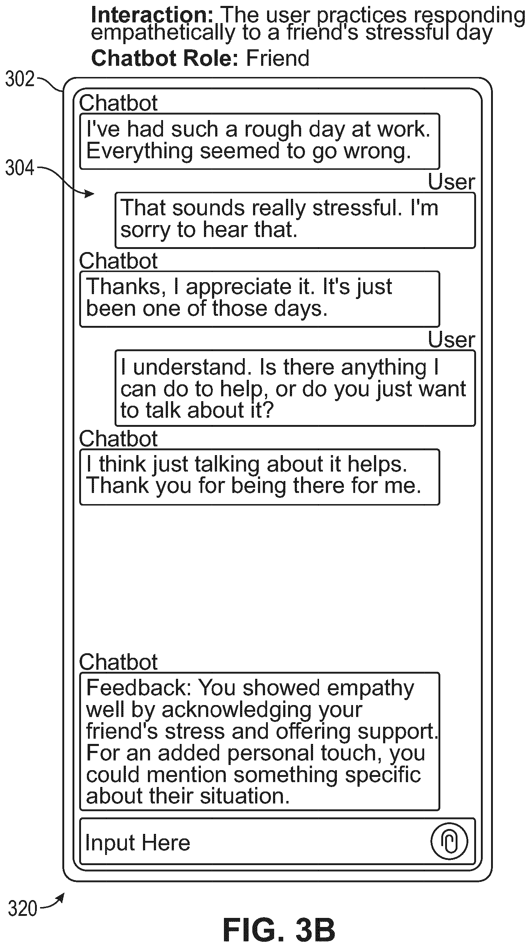

Referring now to FIG. 3B is an example interface for providing personalized digital therapeutics, in accordance with some implementations of the present disclosure. The system 100 interfaces with the digital therapeutic application 304 on the user device 302 to simulate the roleplay interaction 320. Model(s) 107 can be utilized to simulate a conversation where the chatbot takes on the role of a supportive friend. The chatbot begins, "I've had such a rough day at work. Everything seemed to go wrong." The user inputs, "That sounds really stressful. I'm sorry to hear that." The system 100 applies model(s) 107 to process this input and generate the response of the chatbot: "Thanks, I appreciate it. It's just been one of those days." The user continues, "I understand. Is there anything I can do to help, or do you just want to talk about it?" Model(s) 107 generate the reply of the chatbot: "I think just talking about it helps. Thank you for being there for me."

Feedback can be generated by the system 100 through model(s) 107 and provided as, "You showed empathy well by acknowledging your friend's stress and offering support. For an added personal touch, you could mention something specific about their situation." The system 100 evaluates interaction metrics such as empathy demonstrated, contextual relevance, and/or adherence to interaction objectives. Using model(s) 107, the system can refine outputs to ensure consistency with therapeutic goals.

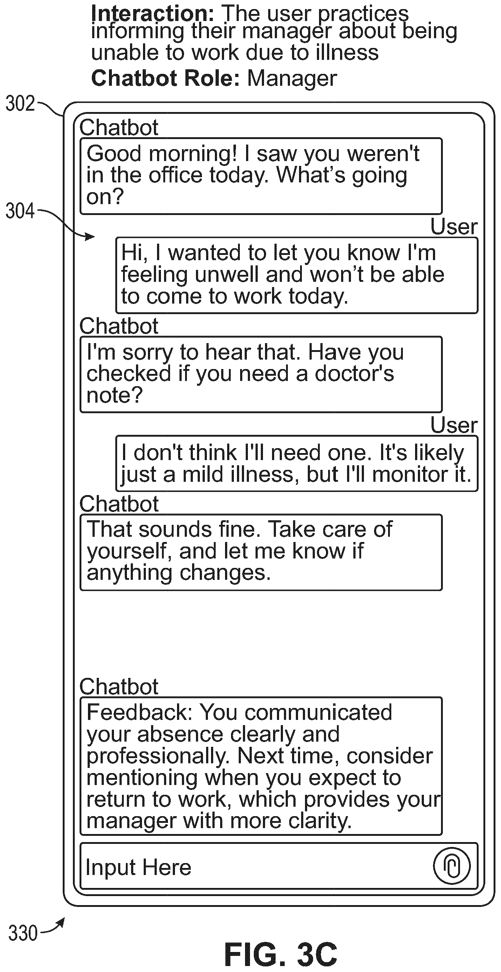

Referring now to FIG. 3C is an example interface for providing personalized digital therapeutics, in accordance with some implementations of the present disclosure. The system 100 interfaces with the digital therapeutic application 304 on the user device 302 to simulate the roleplay interaction 330. This interaction allows the user to practice professionally informing a manager about their condition. The chatbot begins, "Good morning! I saw you weren't in the office today. What's going on?" The user inputs, "Hi, I wanted to let you know I'm feeling unwell and won't be able to come to work today." The system 100 processes this input using model(s) 107 to generate the response of the chatbot: "I'm sorry to hear that. Have you checked if you need a doctor's note?" The user continues, "I don't think I'll need one. It's likely just a mild illness, but I'll monitor it." The chatbot responds with, "That sounds fine. Take care of yourself, and let me know if anything changes."

Feedback can be generated by the system 100 via model(s) 107 and provided through the chatbot as, "You communicated your absence clearly and professionally. Next time, consider mentioning when you expect to return to work, which provides your manager with more clarity." The system 100 can track metrics such as clarity of communication, interaction-specific goals, and/or adherence to professional standards. By using model(s) 107, the system dynamically adjusts chatbot outputs, ensuring relevance to the input of the user.

Various operations described herein can be implemented on computer systems. FIG. 4 shows a simplified block diagram of a representative server system 400, user computer system 414, and network 426 usable to implement certain embodiments of the present disclosure. In various embodiments, server system 400 or similar systems can implement services or servers described herein or portions thereof. System 100 described herein can be like the server system 400. Server system 400 can have a modular design that incorporates a number of modules 402 (e.g., blades in a blade server embodiment); while two modules 402 are shown, any number can be provided. At least one (e.g., each) module 402 can include processing unit(s) 404 and local storage 406.

Processing unit(s) 404 can include a single processor, which can have one or more cores, or multiple processors. In some embodiments, processing unit(s) 404 can include a general-purpose primary processor as well as one or more special-purpose co-processors, such as graphics processors, digital signal processors, or the like. In some embodiments, some or all processing units 404 can be implemented using customized circuits, such as application specific integrated circuits (ASICs) or field programmable gate arrays (FPGAs). In some embodiments, such integrated circuits execute instructions that are stored on the circuit itself. In other embodiments, processing unit(s) 404 can execute instructions stored in local storage 406. Any type of processors in any combination can be included in processing unit(s) 404.

Local storage 406 can include volatile storage media (e.g., DRAM, SRAM, SDRAM, or the like) and/or non-volatile storage media (e.g., magnetic, or optical disk, flash memory, or the like). Storage media incorporated in local storage 406 can be fixed, removable, or upgradeable as desired. Local storage 406 can be physically or logically divided into various subunits such as a system memory, a read-only memory (ROM), and a permanent storage device. The system memory can be a read-and-write memory device or a volatile read-and-write memory, such as dynamic random-access memory. The system memory can store some or all of the instructions and data that processing unit(s) 404 need at runtime. The ROM can store static data and instructions that are needed by processing unit(s) 404. The permanent storage device can be a non-volatile read-and-write memory device that can store instructions and data even when module 402 is powered down. The term "storage medium" as used herein includes any medium in which data can be stored indefinitely (subject to overwriting, electrical disturbance, power loss, or the like) and does not include carrier waves and transitory electronic signals propagating wirelessly or over wired connections.

In some embodiments, local storage 406 can store one or more software programs to be executed by processing unit(s) 404, such as an operating system and/or programs implementing various server functions such as functions of the system 100 or any other system described herein, or any other server(s) associated with system 100 or any other system described herein.

"Software" refers generally to sequences of instructions that, when executed by processing unit(s) 404, cause server system 400 (or portions thereof) to perform various operations, thus defining one or more specific machine embodiments that execute and perform the operations of the software programs. The instructions can be stored as firmware residing in read-only memory and/or program code stored in non-volatile storage media that can be read into volatile working memory for execution by processing unit(s) 404. Software can be implemented as a single program or a collection of separate programs or program modules that interact as desired. From local storage 406 (or non-local storage described below), processing unit(s) 404 can retrieve program instructions to execute and data to process to execute various operations described above.

In some server systems 400, multiple modules 402 can be interconnected via a bus or other interconnect 408, forming a local area network that supports communication between modules 402 and other components of server system 400. Interconnect 408 can be implemented using various technologies, including server racks, hubs, routers, etc.

A wide area network (WAN) interface 410 can provide data communication capability between the local area network (e.g., through the interconnect 408) and the network 426, such as the Internet. Other technologies can be used to communicatively couple the server system with the network 426, including wired (e.g., Ethernet, IEEE 802.3 standards) and/or wireless technologies (e.g., Wi-Fi, IEEE 802.11 standards).

In some embodiments, local storage 406 is intended to provide working memory for processing unit(s) 404, providing fast access to programs and/or data to be processed while reducing traffic on interconnect 408. Storage for larger quantities of data can be provided on the local area network by one or more mass storage subsystems 412 that can be connected to interconnect 408. Mass storage subsystem 412 can be based on magnetic, optical, semiconductor, or other data storage media. Direct attached storage, storage area networks, network-attached storage, and the like can be used. Any data stores or other collections of data described herein as being produced, consumed, or maintained by a service or server can be stored in mass storage subsystem 412. In some embodiments, additional data storage resources can be accessible via WAN interface 410 (potentially with increased latency).

Server system 400 can operate in response to requests received via WAN interface 410. For example, one of modules 402 can implement a supervisory function and assign discrete tasks to other modules 402 in response to received requests. Work allocation techniques can be used. As requests are processed, results can be returned to the requester via WAN interface 410. Such operation can generally be automated. Further, in some embodiments, WAN interface 410 can connect multiple server systems 400 to each other, providing scalable systems capable of managing high volumes of activity. Other techniques for managing server systems and server farms (collections of server systems that cooperate) can be used, including dynamic resource allocation and reallocation.

Server system 400 can interact with various user-owned or user-operated devices via a wide-area network such as the Internet. An example of a user-operated device is shown in FIG. 4 as user computing system 414. User computing system 414 can be implemented, for example, as a consumer device such as a smartphone, other mobile phone, tablet computer, wearable user device (e.g., smart watch, eyeglasses), desktop computer, laptop computer, and so on. For example, user computing system 414 can communicate via WAN interface 420. User computing system 414 can include computer components such as processing unit(s) 416, storage device 418, network interface 420, user input device 422, and user output device 424. User computing system 414 can be a user device implemented in a variety of form factors, such as a desktop computer, laptop computer, tablet computer, smartphone, other mobile user device, wearable user device, or the like.

Processing unit 416 and storage device 418 can be similar to processing unit(s) 404 and local storage 406 described above. Suitable devices can be selected based on the demands to be placed on user computing system 414; for example, user computing system 414 can be implemented as a "thin" user with limited processing capability or as a high-powered user device. User computing system 414 can be provisioned with program code executable by processing unit(s) 416 to enable various interactions with server system 400.

Network interface 420 can provide a connection to the network 426, such as a wide area network (e.g., the Internet) to which WAN interface 410 of server system 400 is also connected. In various embodiments, network interface 420 can include a wired interface (e.g., Ethernet) and/or a wireless interface implementing various RF data communication standards such as Wi-Fi, Bluetooth, or cellular data network standards (e.g., 3G, 4G, LTE, 5G, etc.).

User input device 422 can include any device (or devices) via which a user can provide signals to user computing system 414; user computing system 414 can interpret the signals as indicative of user requests or information. In various embodiments, user input device 422 can include at least one of a keyboard, touch pad, touch screen, mouse, or other pointing device, scroll wheel, click wheel, dial, button, switch, keypad, microphone, and so on.

User output device 424 can include any device via which user computing system 414 can provide information to a user. For example, user output device 424 can include display-to-display images generated by or delivered to user computing system 414. The display can incorporate various image generation technologies, e.g., a liquid crystal display (LCD), light-emitting diode (LED) display including organic light-emitting diodes (OLED), projection system, cathode ray tube (CRT), or the like, together with supporting electronics (e.g., digital-to-analog or analog-to-digital converters, signal processors, or the like). Some embodiments can include a device such as a touchscreen that function as both input and output device. In some embodiments, other user output devices 424 can be provided in addition to or instead of a display. Examples include indicator lights, speakers, tactile "display" devices, printers, and so on.

Some embodiments include electronic components, such as microprocessors, storage, and memory that store computer program instructions in a computer readable storage medium. Many of the features described in this specification can be implemented as processes that are specified as a set of program instructions encoded on a computer readable storage medium. When one or more processing units execute these program instructions, they cause the processing unit(s) to perform various operations indicated in the program instructions. Examples of program instructions or computer code include machine code, such as is produced by a compiler, and files including higher-level code that are executed by a computer, an electronic component, or a microprocessor using an interpreter. Through suitable programming, processing unit(s) 404 and 416 can provide various functionality for server system 400 and user computing system 414, including any of the functionality described herein as being performed by a server or user, or other functionality.

It will be appreciated that server system 400 and user computing system 414 are illustrative and that variations and modifications are possible. Computer systems used in connection with embodiments of the present disclosure can have other capabilities not specifically described here. Further, while server system 400 and user computing system 414 are described with reference to particular blocks, it is to be understood that these blocks are defined for convenience of description and are not intended to imply a particular physical arrangement of component parts. For instance, different blocks can be but need not be in the same facility, in the same server rack, or on the same motherboard. Further, the blocks need not correspond to physically distinct components. Blocks can be configured to perform various operations, e.g., by programming a processor or providing appropriate control circuitry, and various blocks can or cannot be reconfigurable depending on how the initial configuration is obtained. Embodiments of the present disclosure can be realized in a variety of apparatus including electronic devices implemented using any combination of circuitry and software.

While the disclosure has been described with respect to specific embodiments, one skilled in the art will assess that numerous modifications are possible. Embodiments of the disclosure can be realized using a variety of computer systems and communication technologies, including specific examples described herein. Embodiments of the present disclosure can be realized using any combination of dedicated components and/or programmable processors and/or other programmable devices. The various processes described herein can be implemented on the same processor or different processors in any combination. Where components are described as being configured to perform certain operations, such configuration can be accomplished, e.g., by designing electronic circuits to perform the operation, by programming programmable electronic circuits (such as microprocessors) to perform the operation, or any combination thereof. Further, while the embodiments described above can refer to specific hardware and software components, those skilled in the art will appreciate that different combinations of hardware and/or software components can also be used and that particular operations described as being implemented in hardware can also be implemented in software or vice versa.

Computer programs incorporating various features of the present disclosure can be encoded and stored on various computer readable storage media; suitable media include magnetic disk or tape, optical storage media such as compact disk (CD) or digital versatile disk (DVD), flash memory, and other non-transitory media. Computer readable media encoded with the program code can be packaged with a compatible electronic device, or the program code can be provided separately from electronic devices (e.g., via Internet download or as a separately packaged computer-readable storage medium).

Example embodiments will now be set forth in the following enumerated clauses:
1. A system, comprising:
   one or more processors coupled with memory configured to:
      receive, during a session for a roleplay interaction having a defined role for a user and a defined role for a chatbot, content from a user device in the roleplay interaction;
      apply the content as input to at least one artificial intelligence (AI) model to cause the at least one AI model to generate an output for the chatbot based on the defined role for the user, the defined role for the chatbot, and the roleplay interaction; and
      provide, during the session via a chatbot interface, the output to the user device.
2. The system of clause 1, wherein the one or more processors coupled with the memory is further configured to:
   receive a request corresponding with initiating the session;
   identify the roleplay interaction from a plurality of roleplay interactions based at least on data of the user device; and
   initiate the session for the roleplay interaction.
3. The system of clause 1 or clause 2, wherein identifying the roleplay interaction from a plurality of roleplay interactions is based at least on a treatment journey and a condition of the user.
4. The system of any preceding clause, wherein a plurality of roleplay interactions are maintained in a data source and organized based on at least one interaction objective or at least one treatment outcome.
5. The system of any preceding clause, wherein the defined role for the user is based on a condition of the user.
6. The system of any preceding clause, wherein the defined role for the chatbot is based on the roleplay interaction identified during an initiation of the session.
7. The system of any preceding clause, wherein the output corresponds to advancing a treatment journey corresponding with a condition of the user.
8. The system of any preceding clause, wherein the output corresponds to providing a dialogue corresponding with completing an objective of the roleplay interaction or performing an action of the roleplay interaction.
9. The system of any preceding clause, wherein the one or more processors coupled with the memory is further configured to:
   determine a metric of the session based at least on one or more (i) a plurality of user responses, (ii) data of the user device, (iii) content from the user device, (iv) interaction time, (v) completion of an objective of the roleplay interaction, or (vi) performance of an action of the roleplay interaction; and
   update a profile of the user based on the metric.
10. The system of any preceding clause, wherein the one or more processors coupled with the memory is further configured to:
   update, during the session, the defined role for the chatbot based on at least one of (i) a plurality of user responses, (ii) data of the user device, (iii) content from the user device, (iv) interaction time, (v) completion of an objective of the roleplay interaction, or (vi) performance of an action of the roleplay interaction.
11. The system of any preceding clause, wherein the roleplay interaction is personalized to the user based on at least one of:
   data of the user device;
   content from the user device;
   completion of an objective of the roleplay interaction;
   performance of an action of the roleplay interaction;
   session data of the user;
   behavioral indicator of the user;
   demographic data of the user;
   values of the user;
   hobbies of the user;
   cognitive capacity of the user;
   preferences of the user;
   aesthetics of the user;
   reading level of the user;
   language level of the user;
   difficulty level of the user;
   complexity level of the user; or
   language of the user.
12. The system of any preceding clause, wherein the one or more processors coupled with the memory is further configured to:
   apply the output as input to a compliance model to cause the compliance model to validate adherence of the output to at least one parameter of the chatbot.
13. The system of any preceding clause, wherein the output comprises a request for a response by the user device, and wherein the output is based on at least one of (i) performance during the session, (ii) selection of a new roleplay interaction, (iii) feedback generated during the session for the user, or (iv) feedback generated during the session from the user.
14. The system of any preceding clause, wherein the output comprises feedback corresponding to at least one of (i) analysis of user performance or (ii) providing one or more recommendations for completing at least one objective of the roleplay interaction.
15. The system of any preceding clause, wherein the at least one AI model is updated according to training data corresponding with a plurality of roleplay interactions.
16. The system of any preceding clause, wherein the one or more processors coupled with the memory is further configured to:
   receive, during the session, a recording of the user; and
   provide, during the session or after the session, at least a portion of the recording to the user.
17. The system of any preceding clause, wherein the user is on a medication to address a social skill or emotional skill deficit at least in partial concurrence with the session for the roleplay interaction wherein the medication comprises at least one of lithium, valproate, lamotrigine, carbamazepine, lamotrigine, chlorpromazine, fluphenazine, haloperidol, perphenazine, clozapine, olanzapine, ziprasidone, paliperidone, olanzapine, olanzapine, quetiapine, risperidone, aripiprazole, escitalopram, paroxetine, duloxetine, buspirone, fluoxetine, sertraline, venlafaxine, diazepine, lorazepam, alprazolam, clonazepam, nadolol, penbutolol, pindolol, propranolol, sotalol, timolol, acebutolol, esmolol, betaxolol, metoprolol, bisoprolol, labetalol, or carvedilol.
18. A method, comprising:
   receiving, by one or more processors during a session for a roleplay interaction having a defined role for a user and a defined role for a chatbot, content from a user device in the roleplay interaction;
   applying, by the one or more processors, the content as input to at least one artificial intelligence (AI) model to cause the at least one AI model to generate an output for the chatbot based on the defined role for the user, the defined role for the chatbot, and the roleplay interaction; and
   providing, by the one or more processors during the session via a chatbot interface, the output to the user device.
19. The method of clause 18, further comprising:
   receiving, by the one or more processors, a request corresponding with initiating the session;
   identifying, by the one or more processors, the roleplay interaction from a plurality of roleplay interactions based at least on data of the user device; and
   initiating, by the one or more processors, the session for the roleplay interaction.
20. The method of clause 18 or clause 19, wherein identifying the roleplay interaction from a plurality of roleplay interactions is based at least on a treatment journey and a condition of the user.
21. The method of any one of clauses 18 to 20, wherein a plurality of roleplay interactions are maintained in a data source and organized based on at least one interaction objective or at least one treatment outcome.
22. The method of any one of clauses 18 to 21, wherein the defined role for the user is based on a condition of the user.
23. The method of any one of clauses 18 to 22, wherein the defined role for the chatbot is based on the roleplay interaction identified during an initiation of the session.
24. The method of any one of clauses 18 to 23, wherein the output corresponds to advancing a treatment journey corresponding with a condition of the user.

Thus, although the disclosure has been described with respect to specific embodiments, it will be appreciated that the disclosure is intended to cover all modifications and equivalents within the scope of the following claims.

## Claims

1. A method, comprising:
receiving, by one or more processors during a session for a roleplay interaction having a defined role for a user and a defined role for a chatbot, content from a user device in the roleplay interaction;
applying, by the one or more processors, the content as input to at least one artificial intelligence (AI) model to cause the at least one AI model to generate an output for the chatbot based on the defined role for the user, the defined role for the chatbot, and the roleplay interaction; and
providing, by the one or more processors during the session via a chatbot interface, the output to the user device.

2. The method of claim 1, further comprising:
receiving, by the one or more processors, a request corresponding with initiating the session;
identifying, by the one or more processors, the roleplay interaction from a plurality of roleplay interactions based at least on data of the user device; and
initiating, by the one or more processors, the session for the roleplay interaction.

3. The method of claim 1 or claim 2, wherein identifying the roleplay interaction from a plurality of roleplay interactions is based at least on a treatment journey and a condition of the user.

4. The method of any one preceding claim, wherein a plurality of roleplay interactions are maintained in a data source and organized based on at least one interaction objective or at least one treatment outcome.

5. The method of any one preceding claim 1, wherein the defined role for the user is based on a condition of the user, and/or
wherein the defined role for the chatbot is based on the roleplay interaction identified during an initiation of the session.

6. The method of any one preceding claim, wherein the output corresponds to advancing a treatment journey corresponding with a condition of the user, and/or
wherein the output corresponds to providing a dialogue corresponding with completing an objective of the roleplay interaction or performing an action of the roleplay interaction.

7. The method of any one preceding claim, further comprising:
determining, by the one or more processors, a metric of the session based at least on one or more (i) a plurality of user responses, (ii) data of the user device, (iii) content from the user device, (iv) interaction time, (v) completion of an objective of the roleplay interaction, or (vi) performance of an action of the roleplay interaction; and
updating, by the one or more processors, a profile of the user based on the metric.

8. The method of any one preceding claim, further comprising:
update, by the one or more processors, during the session, the defined role for the chatbot based on at least one of (i) a plurality of user responses, (ii) data of the user device, (iii) content from the user device, (iv) interaction time, (v) completion of an objective of the roleplay interaction, or (vi) performance of an action of the roleplay interaction.

9. The method of any one preceding claim, wherein the roleplay interaction is personalized to the user based on at least one of:
data of the user device;
content from the user device;
completion of an objective of the roleplay interaction;
performance of an action of the roleplay interaction;
session data of the user;
behavioral indicator of the user;
demographic data of the user;
values of the user;
hobbies of the user;
cognitive capacity of the user;
preferences of the user;
aesthetics of the user;
reading level of the user;
language level of the user;
difficulty level of the user;
complexity level of the user; or
language of the user.

10. The method of any preceding claim, further comprising:
applying, by the one or more processors, the output as input to a compliance model to cause the compliance model to validate adherence of the output to at least one parameter of the chatbot.

11. The method of any one preceding claim, wherein the output comprises a request for a response by the user device, and wherein the output is based on at least one of (i) performance during the session, (ii) selection of a new roleplay interaction, (iii) feedback generated during the session for the user, or (iv) feedback generated during the session from the user, and/or
wherein the output comprises feedback corresponding to at least one of (i) analysis of user performance or (ii) providing one or more recommendations for completing at least one objective of the roleplay interaction.

12. The method of any one preceding claim, wherein the at least one AI model is updated according to training data corresponding with a plurality of roleplay interactions.

13. The method of any one preceding claim, further comprising:
receiving, by the one or more processors, during the session, a recording of the user; and
providing, by the one or more processors, during the session or after the session, at least a portion of the recording to the user.

14. The method of any one preceding claim, wherein the user is on a medication to address a social skill or emotional skill deficit at least in partial concurrence with the session for the roleplay interaction wherein the medication comprises at least one of lithium, valproate, lamotrigine, carbamazepine, lamotrigine, chlorpromazine, fluphenazine, haloperidol, perphenazine, clozapine, olanzapine, ziprasidone, paliperidone, olanzapine, olanzapine, quetiapine, risperidone, aripiprazole, escitalopram, paroxetine, duloxetine, buspirone, fluoxetine, sertraline, venlafaxine, diazepine, lorazepam, alprazolam, clonazepam, nadolol, penbutolol, pindolol, propranolol, sotalol, timolol, acebutolol, esmolol, betaxolol, metoprolol, bisoprolol, labetalol, or carvedilol.

15. A system, comprising:
one or more processors coupled with memory configured to perform the method of any one preceding claim.
